# EUROPEAN PATENT SPECIFICATION

(11) **EP 1 628 968 B1**
(45) Date of publication and mention of the grant of the patent: **06.04.2011**
(21) Application number: 04728708.1
(22) Date of filing: 21.04.2004
(51) Int. Cl.: C07D 401/04, C07D 401/14, C07D 403/04, C07D 405/14, C07D 409/14, A61K 31/506, A61K 31/4439, A61P 3/10, A61P 7/02, A61P 9/10, A61P 19/00, A61P 25/16, A61P 25/28, A61P 29/00, A61P 37/06

(54) **4-IMIDAZOLIN-2-ONE COMPOUNDS**
4-IMIDAZOLIN-2-ONVERBINDUNGEN
COMPOSES DE 4-IMIDAZOLINE-2-ONE

(30) Priority: 21.04.2003 JP 2003116076
(43) Date of publication of application: 01.03.2006
(73) Proprietor: Mitsubishi Tanabe Pharma Corporation, Chuo-ku Osaka-shi Osaka 541-8505 (JP)
(72) Inventor: KUBO, Akira c/o Tanabe Seiyaku Co., Ltd., Osaka 541-8505 (JP); IMASHIRO, Ritsuo c/o Tanabe Seiyaku Co., Ltd., Osaka 541-8505 (JP); OGASAWARA, Akihito c/o Tanabe Seiyaku Co., Ltd., Osaka 541-8505 (JP); NAKAJIMA, Tatsuo c/o Tanabe Seiyaku Co., Ltd., Osaka 541-8505 (JP); NAKANE, Tetsu c/o Tanabe Seiyaku Co., Ltd., Osaka 541-8505 (JP); MIYOSHI, Hidetaka c/o Tanabe Seiyaku Co., Ltd., Chuo-ku, Osaka-shi, Osaka 541-8505 (JP)
(74) Representative: Duckworth, Timothy John
(86) International application number: PCT/JP2004/005716
(87) International publication number: WO 2004/094404

(56) References cited:
- WO-A1-85/02402
- WO-A1-03/035638
- US-A- 3 538 104
- CARLING R.W. ET AL: '1-(3-cyanobenzylpiperidin-4-yl)-5-methyl-4 -phenyl-1,3-dihydroimidazol-2-one: a selective high-affinity antagonist for the human dopamine D4 receptor with excellent selectivity over ion channels' J. MED. CHEM. vol. 42, no. 14, 1999, pages 2706 - 2715, XP001093697

## Description

### Technical Field

The present invention relates to a novel 4-imidazolin-2-one compound which has an excellent p38MAP kinase inhibitory action and is useful for a medicament.

### Background Art

Mitogen-activated protein (MAP) kinase is a member of serine-threonine kinases which transfers a y -phosphate group of adenosine triphosphate (ATP) to a hydroxy of specific serine or threonine which constitutes a protein, and is involved in various cellular responses against extracellular signals. p38 MAP kinase is an about 38 kDa protein and cloned as a homologue of MAP kinases.

p38MAP kinase is activated by inflammatory cytokines such as tumor necrosis factor α (TNF-α) and interleukin 1 (IL-1) , and by stimulation caused by stress such as ultraviolet irradiation. p38 MAP kinase recognizes various transcription factors and protein kinases as a substrate. It has been clearly shown that, being activated by p38 MAP kinase, these transcription factors and protein kinases become involved in promoting transcription, post-transcriptional regulation (e.g. stabilizing mRNA and promoting protein translation) or stabilizing proteins, etc. of various proteins including inflammatory cytokines, which are involved in inflammatory reactions. From these findings, it is thought that p38 MAP kinase is critically involved in the various inflammatory reactions by regulating the production and the signal transduction of inflammatory cytokines, and an inhibitor of p38 MAP kinase can highly expected to serve as a therapeutic agent for various diseases including inflammatory diseases.

As the inhibitors for p38 MAP kinase, there have been disclosed imidazole derivatives in PCT Japanese Provisional Patent Publication No.2000-503304, 1,3-thiazole derivatives in Japanese Provisional Patent Publication No. 2001-114690, 1,3-thiazole derivatives and 1,3-oxazole derivatives in Japanese Provisional Patent Publication No. 2001-114779, imidazole derivatives, pyrrole derivatives, furan derivatives, 3-pyrazolin-5-one derivatives, pyrazole derivatives and thiophene derivative, etc. in Expert Opinion on Therapeutic Patents (2000) 10(1) : 25-37, respectively. However, there has been no description on 4-imidazolin-2-one derivatives, in an of these. US 3538104 discloses imidazol-2-ones with anti-inflammatory activity.

An object of the present invention is to provide a novel compound having an excellent p38 MAP kinase inhibitory action and is useful as a pharmaceutical.

### Disclosure of the Invention

The present invention provides a compound selected from free base compounds of structures 399 to 478 as defined in claim 1, or pharmaceutically acceptable salt thereof.

The present invention also provides a pharmaceutical composition comprising a compound of the present invention or a pharmaceutically acceptable salt thereof and a pharmaceutically acceptable carrier.

The present invention relates to a method of inhibiting of p38 MAP kinase which comprises administering the compound of the present invention or a pharmaceutically acceptable salt thereof to a human in need thereof.

The present invention also relates to a method of prophylaxis or treatment for diseases related to the activation of p38 MAP kinase or the excessive production of inflammatory mediators concerned with p38 MAP kinase, which comprises administering the compound of the present invention or a pharmaceutically acceptable salt thereof to a human in need thereof.

The present invention relates to a method of prophylaxis or treatment for diseases selected from the group consisting of arthritis, inflammatory bowel disease, inflammatory dermal disease, inflammatory respiratory disease, inflammatory optical disease, nephritis, hepatitis, systemic inflammatory disease, shock, cerebrovascular disease, ischemic cardiac diseases, osteoporosis, multiple sclerosis, diabetes, malignant tumor, cachexia, Alzheimer's disease, Parkinson's disease, acquired immunodeficiency syndrome, arterial sclerosis, disseminated intravascular coagulation syndrome, rejection and graft-versus-host diseases by organ transplantation, which comprises administering the compound of the present invention or a pharmaceutically acceptable salt thereof to a human in need thereof. In particular, the present invention provides a compound of the present invention or a pharmaceutically acceptable salt thereof for use in the prophylaxis or treatment of any one of these diseases, and also provides the use of a compound of the present invention or a pharmaceutically acceptable salt thereof for the preparation of an agent for prophylaxis or treatment of any one of these diseases. As used below, "alkyl" is exemplified by a straight or branched chain C₁-C₆ alkyl, and specifically, methyl, ethyl, propyl, isopropyl, butyl, isobutyl, pentyl, hexyl, etc. Preferred is a c₁-c₄alkyl.

Although an optical isomer based on an asymmetric carbon can be present in the compounds of the present invention and their pharmaceutically acceptable salts, the present invention includes any of these optical isomers as well as mixtures thereof. The compounds of the invention can be used for a pharmaceutical use, in either a free form or in a form of a pharmaceutically acceptable salt. A pharmaceutically acceptable salt of a compound of the present invention is exemplified by an inorganic acid salt such as a hydrochloride, a sulfate, a phosphate and a hydrobromide, and an organic acid salt such as acetate, fumarate, oxalate, citrate, methanesulfonate, benzenesulfonate, tosylate and maleate, etc. Further, in case of having a substituent such as carboxy, etc., there are mentioned a salt with a base (for example, an alkali metal salt such as a sodium salt, a potassium salt, etc. and an alkaline earth metal such as a calcium salt) .

The compounds of the present invention include an internal salt and a solvate, such as a hydrate, of a structure as defined in claim 1.

The compounds of the present invention or a pharmaceutically acceptable salt thereof have an excellent p38 MAP kinase inhibitory action and are useful for the prophylaxis and treatment for diseases related to the activation of p38 MAP kinase and the excessive production of inflammatory mediators concerned with p38 MAP kinase such as TNF-α, IL-1, etc. Therefore, the compounds of the present invention or a pharmaceutically acceptable salt thereof is expected to be useful for a therapeutic and prophylactic agent for inflammatory diseases, etc. such as arthritis (rheumatoid arthritis, osteoarthritis, infectious arthritis, gouty arthritis, traumatic arthritis, synovitis, periarthritis, etc.), inflammatory bowel disease (ulcerative colitis, Crohn's disease, etc.), inflammatory dermal disease' [psoriasis, dermatitis (atopic dermatitis, contact dermatitis , urticaria, eczema, etc.), etc.], inflammatory respiratory disease (asthma, bronchitis, pneumonia, pleurisy, pharyngitis, rhinitis, etc.), inflammatory optical disease (conjunctivitis, keratitis, uveitis, etc.), nephritis, hepatitis, systemic inflammatory disease (Behcet's syndrome, systemic lupus erythematosus, etc.), shock (septic shock, endotoxin shock, etc.), cerebrovascular disease (cerebral hemorrhage, cerebral infarction, cerebral edema, etc.), ischemic cardiac diseases (angina pectoris, cardiac infarction, congestive heart failure, etc.), osteoporosis, multiple sclerosis, diabetes, malignant tumor, cachexia, Alzheimer's disease, Parkinson's disease, acquired immunodeficiency syndrome, arterial sclerosis, disseminated intravascular coagulation syndrome, rejection and graft-versus-host diseases by organ transplantation, etc.

The compound of the present invention can be used in combination with one or more drugs selected from the group consisting of non-steroidal anti-inflammatory drugs, anti-rheumatic drugs, anti-cytokine drugs, immunosuppressants and steroids.

Examples of the non-steroidal anti-inflammatory drug include alcofenac, aceclofenac, sulindac, tolmetin, fenoprofen, thiaprofenic acid, tenoxicam, lornoxicam, aspirin, mefenamic acid, flufenamic acid, diclofenac, loxoprofen, phenylbutazone, indomethacin, ibuprofen, ketoprofen, naproxen, flurbiprofen, pranoprofen, piroxicam, zaltoprofen, celecoxib, rofecoxib, valdecoxib, salts thereof and the like.

Examples of the anti-rheumatic drug include gold preparation (Auranofin, etc.), penicillamine, bucillamine, lobenzarit, actarit, sulfasalazine, chloroquine, leflunomide, and the like.

Examples of the anti-cytokine drug include etanercept, infliximab, soluble TNF-α receptor, anti-TNF-α antibody, anti-interleukin-6 antibody, anti-interleukin-12 antibody and the like.

Examples of the immunosuppressant include methotrexate, cyclophosphamide, brequinar sodium, deoxyspergualin, mizoribine, 2-morphorinoethyl mycophenolate, rimexolone, cyclosporine, rapamycin, tacrolimus, gusperimus, azathiopurine and the like.

Examples of the steroid include dexamethasone, betamethasone, triamcinolone, fluocinonide, prednisolone, methylprednisolone, cortisone acetate, hydrocortisone and the like.

When the compound of the present invention or pharmaceutically acceptable salt thereof is used in combination with one or more drugs above, two or more ingredients can be administered simultaneously, subsequently or separately with intervals.

The compound of the present invention or a pharmaceutically acceptable salt thereof can be formulated into a pharmaceutical composition comprising a therapeutically effective amount of a compound of the present invention and a pharmaceutically acceptable carrier therefor. The pharmaceutically acceptable carriers include diluents, binders (e.g., syrup, gum arabic, gelatine, sorbit, tragacanth, polyvinylpyrrolidone), excipients (e.g., lactose, sucrose, corn starch, potassium phosphate, sorbit, glycine), lubricants (e.g., magnesium stearate, talc, polyethylene glycol, silica), disintegrants (e.g., potato starch) and wetting agents (e.g., sodium lauryl sulfate), and the like.

The compound of the present invention or a pharmaceutically acceptable salt thereof can be administered orally or parenterally, and be used as an appropriate pharmaceutical preparation. Examples of an appropriate preparation for oral administration include solid preparations (tablets, granules, capsules, powders, etc.), solutions, suspensions and emulsions. Examples of an appropriate preparation for parenteral administration include suppository, injections or preparation for continuous infusion prepared using distilled water for injection, physiological saline or aqueous glucose solution, etc., or inhalant.

An administration amount of the compound of the present invention or a pharmaceutically acceptable salt thereof depends on an administration method, age, body weight, and condition of the patient, and usually, it is preferably 0.003 to 30 mg/kg, and particularly preferably, 0.01 to 10 mg/kg.

The compounds of the present invention and their pharmaceutically acceptable salts can be prepared suitably by a method selected from the following [Method A] to [Method D], however, it is not limited to these. Production method will be described in detail using the compound [Ia'] as follows.

### [Method A]

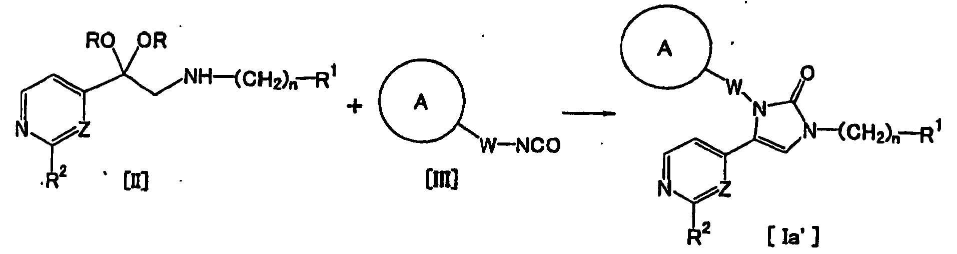

(wherein R is an alkyl, A-W- is 4-fluorophenyl, n is 0, Z is N or CH, and R¹ and R² have the same meanings as mentioned in claim 1)

The compound [Ia'] of the present invention can be produced by reacting a compound [II] with a compound [III], followed by treating the reaction product with an acid. This reaction can be carried out in a solvent (Journal of Medicinal Chemistry, 9, 858 (1966)). As the solvent, there is no limitation as long as it does not affect the reaction, for example, there are mentioned tetrahydrofuran (THF), chloroform, methylene chloride, dioxane, ethyl acetate, ether, toluene, etc. The present reaction proceeds preferably at -20 to 80°C, particularly preferably at 0 to 30°C. Further, as an acid for an acid treatment, there are mentioned, for example, hydrochloric acid, sulfuric acid, phosphoric acid, p-toluenesulfonic acid, methanesulfonic acid, etc. Additionally, as an alkyl of R in the formula [II], there are mentioned, for example, methyl, ethyl, propyl, butyl, etc., and particularly preferred are methyl and ethyl.

### [Method B]

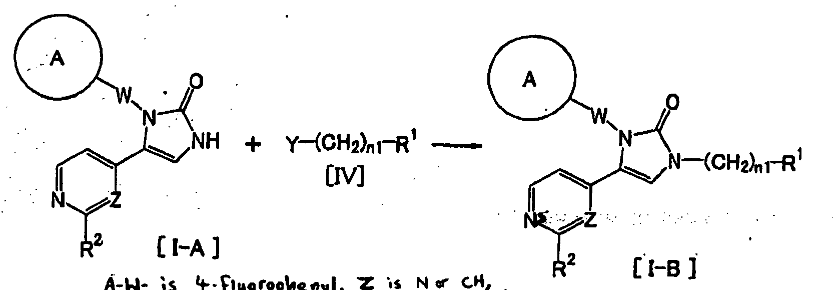

(wherein A-W- is 4-fluorophenyl, Z is N or CH, Y is a halogen atom, hydroxy, or dihydroxyboranyl, n1 is 0, and R¹ and R² have the same meanings as in claim 1)

The compound [I-B] which is categorized in the compound [Ia'] can be produced by reacting a compound [I-A]; with a compound [IV] for alkylation.

When Y in the formula [IV] is a halogen atom, this reaction can be carried out in a solvent, in the presence of a base. Any solvent can be used as long as it does not affect the reaction, and there are mentioned, for example, dimethylformamide (DMF), dimethylsulfoxide,1-methylpyrrolidone, 1,3,-dimethyl-2-imidazolidinone, etc. As the base, there are mentioned, for example, sodium hydride, sodium hydroxide, potassium t-butoxide, butyllithium, lithium diisopropylamide, etc. The reaction proceeds preferably at -20 to 100°C, particularly preferably at 0 to 30°C. Further, as the halogen atom at Y, there are mentioned chlorine, bromine and iodine, and bromine and iodine are particularly preferred.

When Y in the formula [IV] is hydroxy, the reaction can be carried out in a solvent, in the presence of an additive and an activator (Synthesis, 1 (1981)). Any solvent can be used as long as it does not affect the reaction, and there are mentioned, for example, methylene chloride, THF, dioxane, chloroform, etc. As the additive, there are mentioned, for example, triphenylphosphine, tributylphosphine, trimethylphosphine, etc. As the activator, there are mentioned, for example, diethyl azodicarboxylate, dimethyl azodicarboxylate, 1,1-azobis(N,N-dimethylformamide), 1,1-(azodicarbonyl)dipiperidine, etc. This reaction proceeds preferably at -30 to 100°C, and particularly preferably at 0 to 50°C.

When Y in the formula [IV] is dihydroxyboranyl, the reaction can be carried out in a solvent, in the presence of a catalyst and a base (Tetrahedron Letters, 39, 2933 (1998)) Any solvent can be used as long as it does not affect the reaction, and there are mentioned, for example, methylene chloride, chloroform, DMF, etc. As the catalyst, there are mentioned, for example, copper (II) acetate, etc. As the base, there are mentioned, for example, triethylamine, diisopropylethylamine, 4-methylmorpholine, pyridine, etc. This reaction proceeds preferably at -10 to 100°C, and particularly preferably at 20 to 60°C.

### [Method C]

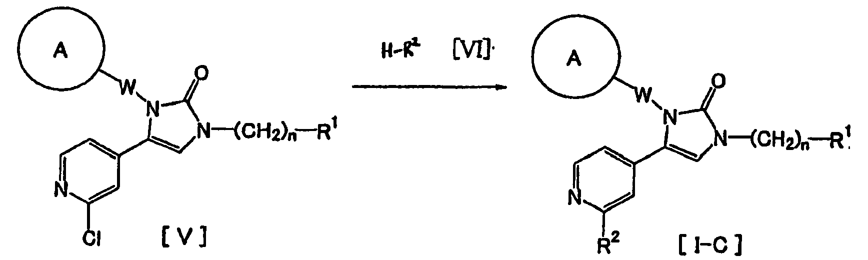

(wherein W-A- is 4-fluorophenyl, n is 0 and R¹ and R² have the same meanings as in structure 477 or 478 as definded in claim 1).

The compound [I-C] can be produced by reacting a compound [V] with a compound [VI].

The reaction between the compound [V] and the compound [VI] can be carried out in a solvent, in the presence of a catalyst, a base and an additive (Journal of Organic Chemistry, 61, 7240 (1996)). Any solvent can be used as long as it does not affect the redaction, and there are mentioned, for example, toluene, xylene, dimethoxyethane, dioxane, etc.

As the catalyst, there are mentioned, for example, palladium acetate, bis (dibenzylideneacetone) dipalladium, etc. As the base, there are mentioned, for example, sodium t-butoxide, potassium t-butoxide, lithium t-butoxide, triethylamine, etc. As the additive, there are mentioned, for example, 2,2'-bis(diphenylphosphino)-1,1'binaphthyl, etc. The reaction proceeds preferably at 30 to 150°C, and particularly preferably at 60 to 80°C.

### [Method D]

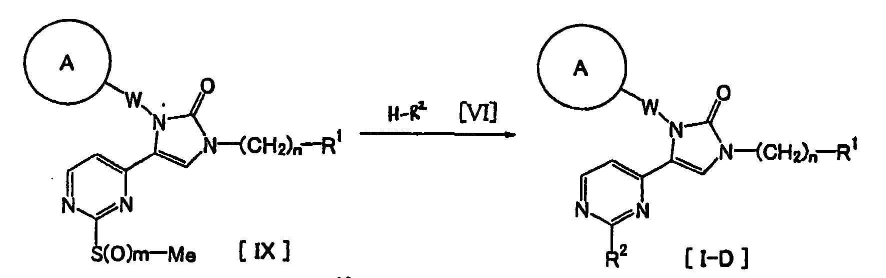

(wherein m is 1 or 2, W-A- is 4-fluorophenyl, n is 0 and R¹ and R² have the same meanings as in structures 399 to 476 as defined in claim 1).

The compound [I-D] can be produced by reacting a compound [IX] with a compound [VI].

The reaction between the compound [IX] and the compound [VI] can be carried out in a solvent. Any solvent can be used as long as it does not affect the reaction, and there are mentioned, for example, dioxane, THF, DMF, dimethylsulfoxide, etc. The reaction proceeds preferably at 0 to 150 °C, and particularly preferably at 50 to 100°C.

The compound [Ia'] produced above can also be derived to other compounds [Ia' ] by converting a functional group using properly a conventionally known organic chemistry reaction. Such a method for converting a functional group may be suitably selected depending on a kind of a desired functional group.

The compound of the present invention obtained according to the above described [Method A] to [Method D] can be optionally converted to a pharmaceutically acceptable salt. Conversion to a pharmaceutically acceptable salt may be carried out by methods known to the person skilled in the art.

In the following, production methods for starting materials used in the above methods are described.

The starting material [II] can be produced as follows. 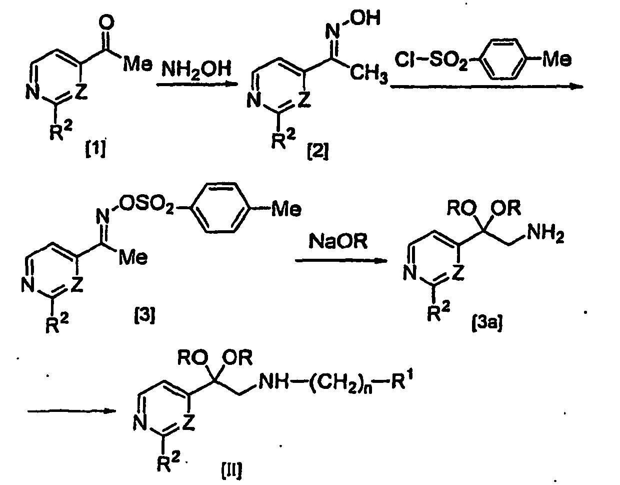 (wherein the symbols have the same meanings as the above.)

The reaction for producing the compound [2] from the compound [1] and hydroxylamine can be carried out in a solvent. Any solvent can be used as long as it does not affect the reaction, and there are mentioned, for example, ethanol, methanol, etc. The reaction proceeds preferably at 0 to 150°C, and particularly preferably at 60 to 80°C.

The reaction for producing the compound [3] from the compound [2] and tosyl chloride can be carried out in a solvent. Any solvent can be used as long as it does not affect the reaction, and there are mentioned, for example, methylene chloride, chloroform, THF, toluene, etc. As the base, there are mentioned, for example, triethylamine, diisopropylethylamine, pyridine, etc. The reaction proceeds preferably at -20 to 80°C, and particularly preferably at 0 to 30°C.

The reaction for producing the compound [3a] from the compound [3] can be carried out in a solvent, by reacting the compound [3] with sodium alkoxide, followed by treating the reactant with an acid. Any solvent can be used as long as it does not affect the reaction, and there are mentioned, for example, methanol, ethanol, dioxane, THF, dimethoxyethane, etc. As the acid, there are mentioned, for example, hydrogen chloride, etc. The reaction proceeds preferably at -20 to 60°C, and particularly preferably at 0 to 30°C.

The reaction for producing the compound [II] from the compound [3a] can be carried out by reacting a corresponding aldehyde using a conventional reductive alkylation (Journal of Organic Chemistry, 61, 3849(1996)).

A starting material [V] can be produced, for example, as follows. 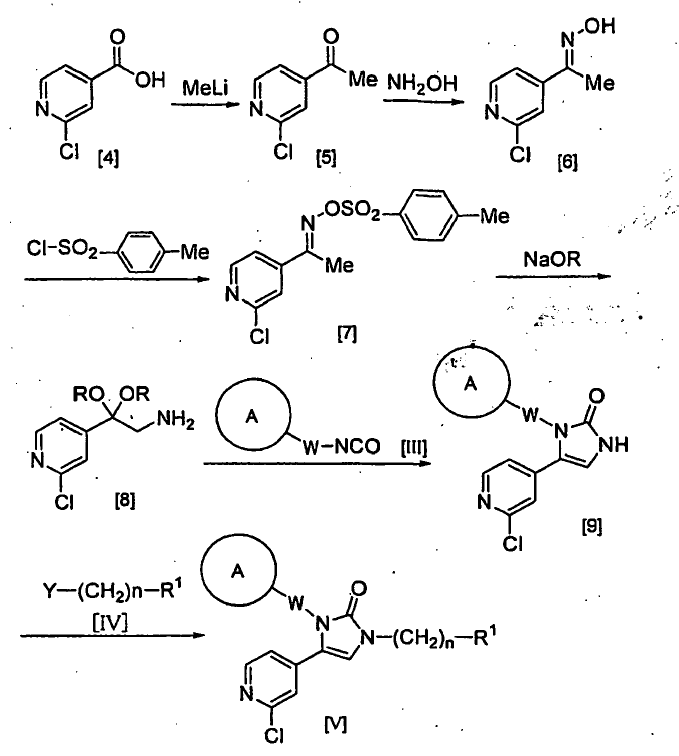 (wherein the symbols have the same meanings as the above.)

The reaction for producing the compound [5] from the compound [4] and methyl lithium can be carried out in a solvent. Any solvent can be used as long as it does not affect the reaction, and there are mentioned, for example, THF, diethyl ether, dimethoxyethane, etc. The reaction proceeds preferably at -90 to 0°C, and particularly preferably at -60 to -40°C.

The method for producing the compound [8] from the compound [5] via the compound [6] and the compound [7] can be carried out in a similar manner to the above-mentioned method for producing the compound [II] from the compound [1] via the compound [2] and the compound [3].

The reaction for producing the compound [9] from the compound [8] and the compound [III] can be carried out in a similar manner to the above-mentioned [Method A].

The reaction for producing the compound [V] from the compound [9] and the compound [IV] can be carried out in a similar manner to the above-mentioned [Method B].

A starting material [IX] can be produced, for example, as follows. 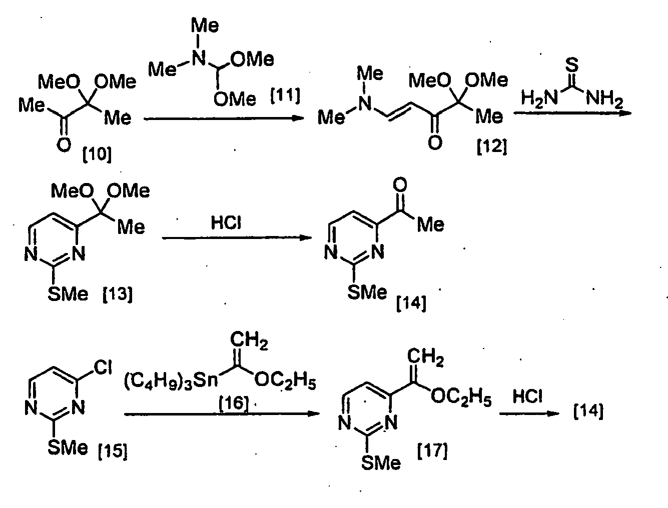 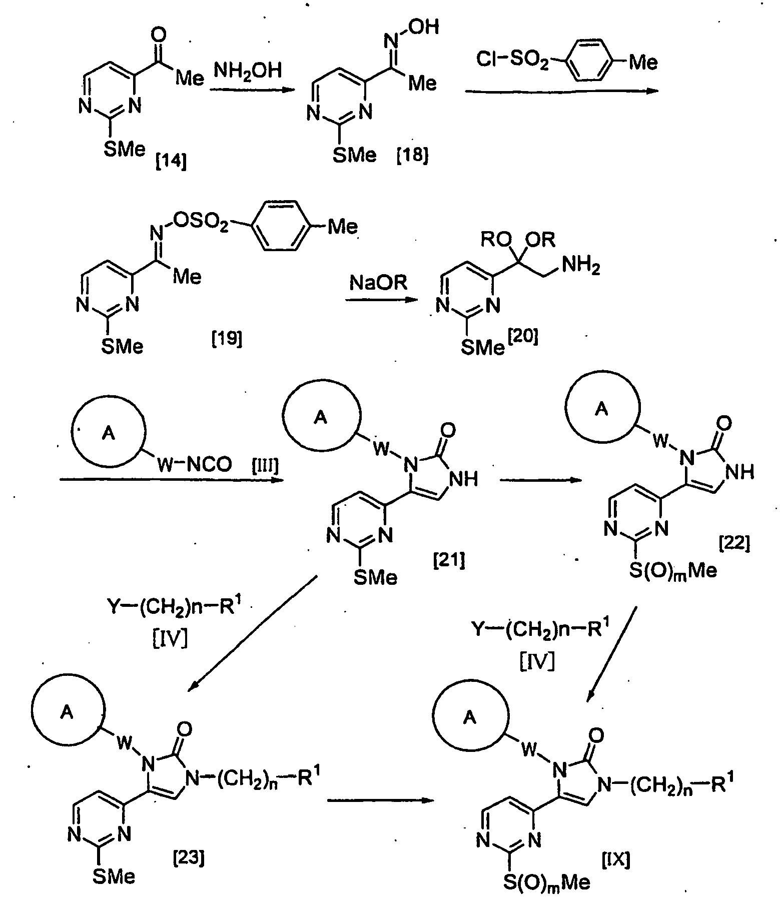 (wherein m is 1 or 2, and other symbols have the same meanings as the above.)

The reaction for producing the compound [12] from the compound [10] and the compound [11] can be carried out in a solvent or without solvent. Any solvent can be used as long as it does not affect the reaction, and there are mentioned, for example, toluene, xylene, dioxane, etc. The reaction proceeds preferably at 50 to 150°C, and particularly preferably at 80 to 120°C.

The reaction for producing the compound [13] from the compound [12] can be carried out by reacting the compound [12] with thiourea in a solvent, in the presence of a base, and then, by reacting an alkylating agent. Any solvent can be used as long as it does not affect the reaction, and there are mentioned, for example, methanol, THF, dioxane, etc. As the base, there are mentioned, for example, sodium methoxide, sodium hydroxide, potassium t-butoxide, etc. As the alkylating agent, there are mentioned, for example, methyl iodide, dimethyl sulfate, etc. The reaction proceeds preferably at 0 to 100°C, and particularly preferably at 30 to 70°C.

The reaction for producing the compound [14] from the compound [13] can be carried out in a solvent, in the presence of an acid. Any solvent can be used as long as it does not affect the reaction, and there are mentioned, for example, water, acetone, THF, dioxane, etc. As the acid, there are mentioned, for example, hydrochloric acid, sulfuric acid, phosphoric acid, p-toluenesulfonic acid, etc. The reaction proceeds preferably at -10 to 80°C, and particularly preferably at 0 to 30°C.

The compound [14] can be also produced from the compound [15] via the compound [17].

The reaction for producing the compound [17] from the compound [15] and the compound [16] can be carried out in a solvent, in the presence of a catalyst. Any solvent.can be used as long as it does not affect the reaction, and there are mentioned, for example, DMF, toluene, xylene, etc. As the catalyst, there are mentioned, for example, bis(triphenylphosphine)palladium dichloride, tetrakis(triphenylphosphine)palladium, etc. The reaction proceeds preferably at 50 to 150°C, and particularly preferably at 70 to 90°C.

The reaction for producing the compound [14] from the compound [17] can be carried out in a similar manner to the above-mentioned method for producing the compound [14] from the compound [13].

The reaction for producing the compound [20] from the compound [14] via the compound [18] and the compound [19]'can be carried out in a similar manner to the above-mentioned method for producing the compound [II] from the compound [1] via the compound [2] and the compound [3].

The reaction for producing the compound [21] from the compound [20] and the compound [III] can be carried out in a similar manner to the above-mentioned [Method A].

The reaction for producing the compound [22] from the compound [21] can be carried out in a solvent, using an oxidizing agent. Any solvent can be used as long as it does not affect the reaction, and there are mentioned, for example, water, methanol, THF, dioxane, chloroform, methylene chloride, etc. As the oxidizing agent, there are mentioned, for example, Oxon (trade name, manufactured by DuPont Co. Ltd.), 3-chloroperoxybenzoic acid, hydrogen peroxide, etc. The reaction proceeds preferably at -20 to 60°C, and particularly preferably at -10 to 30°C.

The reaction for producing the compound [IX] from the compound [22] and the compound [IV] can be carried out in a similar manner to the above-mentioned [Method B].

The compound [IX] can be also produced from the compound [21] via the compound [23].

The reaction for producing the compound [23] from the compound [21] and the compound [IV] can be carried out in a similar manner to the above-mentioned [Method B].

The reaction for producing the compound [IX] from the compound [23] can be carried out in a similar manner to the reaction for producing the compound [22] from the compound [21] .

Incidentally, in the above production methods, it is possible to optionally protect or deprotect a functional group. As the protecting group for the functional group, those used in a field of conventional organic synthetic chemistry can be used, examples of which include those described in "Protective Groups in Organic Synthesis" by T. W. Greene, P. M. G. Wuts, (published by John Wiley and Sons, 1991). For conditions for introducing protecting groups or condition for de-protection, the method described in the above reference can be mentioned.

Further, each compound and each intermediate produced in the above production methods can be purified by means of a conventional method, for example, column chromatography, recrystallization, etc. As a solvent for recrystallization, there are mentioned, for example, an alcohol solvent such as methanol, ethanol, 2-propanol, etc., an ether solvent such as diethyl ether, etc., an ester solvent such as ethyl acetate, etc., an aromatic solvent such as toluene, etc., a ketone solvent such as acetone, etc., a hydrocarbon solvent such as hexane, etc., water, etc., and a mixed solvent thereof. Further, the compounds of the present invention that are free bases can be converted to a pharmaceutically acceptable salt according to the conventional method, and recrystallization can be carried out afterwards.

### Examples

Hereinbelow, the present invention will be explained in more detail with reference to the following Examples, which should not be construed as limiting the scope of the present invention.

Each of the following symbols used in the present specification represents the meaning as described below.
Me : methyl
Et : ethyl
THF: tetrahydrofuran
DMF: N,N-dimethylformamide
t- : tert-

### Example 1 (a reference Example)

### 1-(4-Fluorophenyl)-5-(pyridin-4-yl)-4-imidazolin-2-one

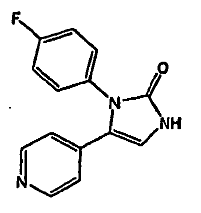

A solution of 3.00 g of 2,2-diethoxy-2-pyridin-4-ylethylamine (a compound obtained in Reference Example 2) dissolved in 30 ml of THF was cooled by water, and 1.97 g of 4-fluorophenylisocyanate was added by dropwise. After addition, the reaction mixture was concentrated under reduced pressure, and then, 30 ml of conc. hydrochloric acid was added to the obtained residue, and the mixture was stirred at room temperature overnight. To 180 ml of an ice cold aqueous 2N NaOH solution was added the reaction mixture for neutralization, and precipitated crystals were collected by filtration. They were washed with water and ether, air-dried at 60°C, to give 3.10 g of the title compound as colorless crystals. Melting point: 261°C (decomposed) Example 2 (a reference Example) 1-Cyclopentylmethyl-3-(9-fluorophenyl)-4-(pyridin-4-yl)-4-imidazolin-2-one · hydrochloride 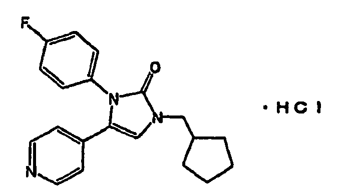 128 mg of 1-(4-Fluorophenyl)-5-(pyridin-4-yl)-4-imidazolin-2-one (the compound of Example 1), 61 µl of cyclopentylmethanol, 197 mg of triphenylphosphine and 295 µl of diethyl azodicarboxylate were dissolved in 2.5 ml of methylene chloride, and the mixture was stirred at room temperature for 24 hours. The reaction mixture was concentrated under reduced pressure, and the residue was purified by silica gel column chromatography (chloroform : ethyl acetate = 19 : 1) . The obtained compound was treated with hydrochloric acid, to give 75 mg of the title compound as powder.

### Example 4 (a reference Example)

### 1-(2-Cyanobenzyl)-3-(4-fluorophenyl)-4-[(2-(1-(S)-phenyl-ethylamino)pyridin-4-yl)]-4-imidazolin-2-one

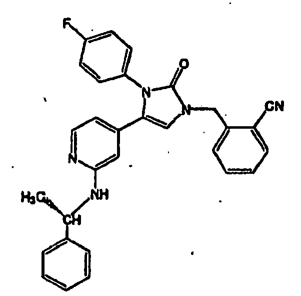

50 mg of 4-(2-Chloropyridin-4-yl)-3-(4-fluorophenyl)-1-(2-cyanobenzy 1) -4-imidazolin-2-one (a compound of Reference Example 1 (6)) , 79 µl of (S)-(-)-α-methylbenzylamine, 5.5 mg of palladium acetate, 15 mg of 2,2'-bis (diphenylphsophino)-1,1'-binaphthyl and 17 mg of sodium t-butoxide were suspended in 1 ml of toluene, and the mixture was stirred at 70°C for 18 hours, under nitrogen flow. The reaction mixture was diluted by ethyl acetate, and insoluble matter was removed by filtration through Celite. To the filtrate was added 6N hydrochloric acid, and after separation, an aqueous layer was made alkaline with aqueous sodium bicarbonate solution. The mixture was extracted with chloroform, washed with saturated brine, and dried over anhydrous magnesium sulfate. After concentration under reduced pressure, the residue was purified by silica gel column chromatography (hexane : ethyl acetate = 1 : 2) , to give 38 mg of the title compound as colorless powder.

### Example 5 (a reference Example)

The compound in Table 1 was obtained by treating the corresponding starting material in a similar manner to that in Example 4.

**Table 1**

| | | | |
|---|---|---|---|
| 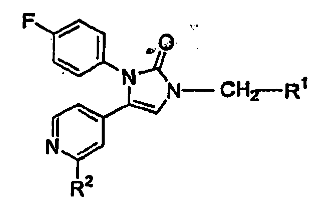 | | | |

| Example | R¹ | R² | Physical properties, etc. |
|---|---|---|---|
| 5 | 2-Cyanophenyl | 4-Methoxybenzylamino | Melting point 167°C |

### Example 13 (a reference Example)

### 4-(2-Aminopyridin-9-yl)-1-(2-cyanobenzyl)-3-(4-fluorophenyl)-4-imidazolin-2-one

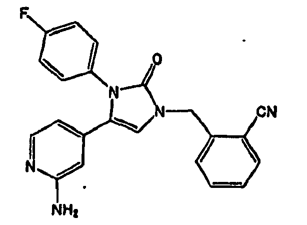

To 1.5 g of 1-(2-cyanobenzyl) -3- (4-fluorophenyl) -4- [2-(4-methoxybenzylamino)pyridin-4-yl]-4-imidazolin-2-one (Compound of Example 5) was added 3 ml of 25% hydrogen bromide-acetic acid solution, and the mixture was stirred at 70°C for one hour. The reaction mixture was concentrated under reduced pressure, and the residue was made alkali with an aqueous sodium bicarbonate solution. The mixture was extracted with ethyl acetate, washed with brine and dried over anhydrous magnesium sulfate. After concentration under reduced pressure, the residue was purified by silica gel column chromatography (chloroform:methanol=20:1) to give 572 mg of the title compound as colorless crystal. Melting point:182-183°C.

### Example 14 (a reference Example)

### 4-(2-N-Isobutyroylaminopyridin-4-yl)-1-(2-cyanobenzyl)-3-(4-fluorophenyl)-4-imidazolin-2-one

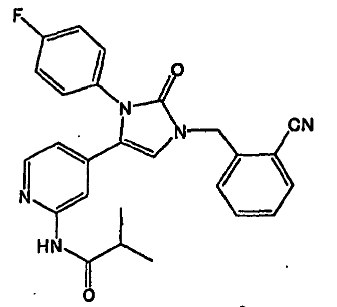

### Example 15 (a reference Example)

### 4-(2-N,N-Diisobutyroylaminopyridin-4-yl)-1-(2-cyanobenzyl)-3-(4-fluorophenyl)-4-imidazolin-2-one

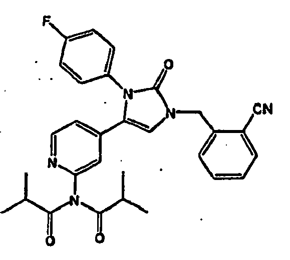

A suspension of 50 mg of 4-(2-aminopyridin-4-yl)-1-(2-cyanobenzyl)-3-(4-fluorophenyl)-4-imidazolin-2-one (Compound of Example 13) and 20 µl of isobutyroyl chloride in methylene chloride was ice-cooled, and after adding 54 µl of triethylamine by dropwise, and the mixture was stirred at room temperature for 3 hours. Water was added to the reaction mixture, and the mixture was extracted with ethyl acetate, washed with brine and dried over anhydrous magnesium sulfate. After concentration under reduced pressure, the residue was purified by silica gel column chromatography (chloroform: acetone=20:1) to give 22 mg of the title compound (Example 14) as colorless crystal and 10 mg of the title compound (Example 15) as colorless crystal, respectively. Melting point:196°C (Example 14), 185-187°C (Example 15).

### Example 17 (a reference Example)

### 1-(2-Cyanobenzyl)-3-(4-fluorophenyl)-4-[2-(3-hydroxypropyl-amino)pyrimidin-4-yl]-4-imidazolin-2-one

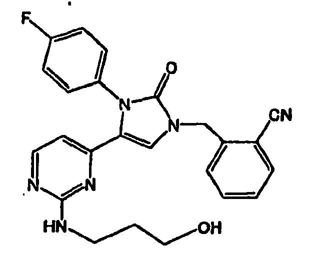

A mixture of 70 mg of 1-(2-cyanobenzyl)-3-(4-fluorophenyl)-4-(2-methylsulfinylpyrimidin-4-yl)-4-imidazolin-2-one (Compound of Reference example 6 (2) or Reference example 7 (2)) , 60.6 mg of 3-aminopropanol and 2 ml of dioxane was stirred at 80°C for 5 hours. The reaction mixture was concentrated and then purified by silica gel column chromatography (chloroform: methanol=19:1) and crystallized from ether to give 44.6 mg of the title compound. Melting point: 166-167°C.

### Examples 55, 57, 61 and 63 (reference Examples)

The compound of Reference example 1(5) and the corresponding starting materials were subjected to N-alkylation in the same manner as in Example 2 or Reference example 1(6), and then, subjected to amination in the same manner as in Example 4 to give the compounds shown in Table 5.

**Table 5**

| | | | |
|---|---|---|---|
| 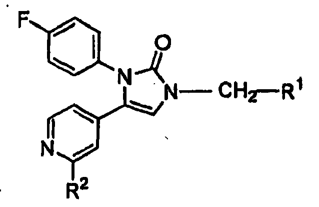 | | | |

| Example | R¹ | R² | MS ([M+H]⁺) |
|---|---|---|---|
| 55 | 4-Methoxyphenyl | Isopropylamino | 433 |
| 57 | 4-Methoxyphenyl | (S)-1-(2-Pyridyl)ethyl-amino | 495 |
| 61 | cis-4-Methoxy-methoxycyclohexyl | Isopropylamino | 469 |
| 63 | 2-Fluorophenyl | (1-t-Butoxycarbonyl-4-pi peridyl)amino | 562 |

### Example 80 (a reference Example)

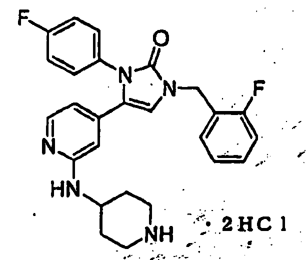

To 146 mg of compound in Example 63 were added 0.2 ml of ethyl acetate and 1.7 ml of a 4N hydrogen chloride-ethyl acetate solution, and the mixture was stirred at room temperature for 3 hours. The reaction mixture was concentrated under reduced pressure, and ethyl acetate was added to the residue and powder was collected by filtration to give 128 mg of the title dompound.
MS 462 ([M+H]⁺)

### Example 81 (a reference Example)

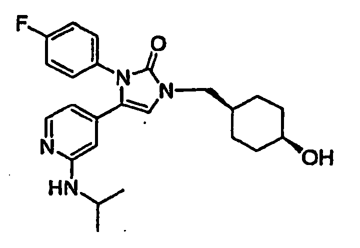

To 2 ml of methanol was dissolved 148 mg of the compound in Example 61, 1 ml of conc. hydrochloric acid was added to the mixture and the resulting mixture was stirred at room temperature overnight. The reaction mixture was concentrated under reduced pressure, and the residue was neutralized with a 4N aqueous NaOH solution and extracted with chloroform. After drying and concentration, diethyl ether and diisopropyl ether were added to the residue and the resulting powder was collected by filtration to give 58 mg of the title compound.
MS 425 ([M+H]⁺)

### Example 147 (a reference Example)

The compound of Reference example 11 and.a corresponding starting compound were subjected to N-alkylation in the same manner as in Reference example 8, and then, the resulting compound was treated with a corresponding isocyanate to carry out cyclization in the same manner as in Example 1 to give the compound shown in Table 14.

**Table 14**

| | | |
|---|---|---|
| 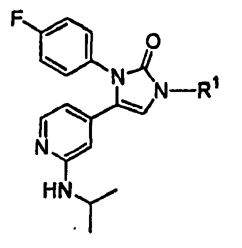 | | |

| Example | R¹ | MS ([M+H]⁺). |
|---|---|---|
| 147* | 4-Piperidyl | 396 |

| | | |
|---|---|---|
| *:Monohydrochloride | | |

### Example 179 (a reference Example)

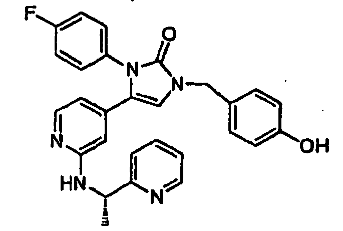

To 5 ml of 25% HBr-acetic acid solution was added 490 mg of the compound of Example 57, and the mixture was stirred at 70°C for 15 hours. After cooling the reaction mixture, an aqueous sodium bicarbonate solution was added to neutralize the mixture, and the resulting mixture was extracted with ethyl acetate, washed with brine and dried over magnesium sulfate. After concentration under reduced pressure, the residue was purified by silica gel column chromatography to give 237 mg of the title compound as colorless powder.
MS 482 ([M+H]⁺)

### Example 180 (a reference Example)

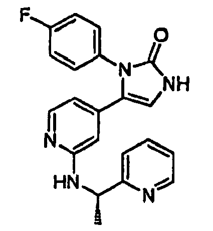

To 200 mg of the compound of Example 179 was added 2 ml of 25% HBr-acetic acid solution, and the mixture was stirred under heating at 80°C for 3 days. After cooling the reaction mixture, an aqueous sodium bicarbonate solution was added thereto to make alkaline, and the mixture was extracted with ethyl acetate, washed with.brine and dried over magnesium sulfate. After concentration under reduced pressure, the residue was purified by silica gel column chromatography to give 71 mg of the title compound as colorless powder.
MS 376 ([M+H]⁺)

### Examples 181 and 182 (reference Examples)

By using the compound of Example 55, it was reacted in the same manner as in Examples 179 and 180 to give the compounds of Examples 181 and 182 shown in Table 20.

**Table 20**

| | | | | |
|---|---|---|---|---|
| 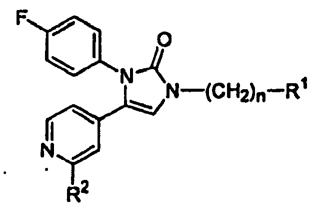 | | | | |

| Example | R¹ | n | R² | MS ([M+H]⁺) |
|---|---|---|---|---|
| 181 | 4-Hydroxyphenyl | 1 | Isopropylamino | 419 |
| 182 | Hydrogen atom | 0 | Isopropylamino | 313 |

### Example 192 (a reference Example)

The compound of Reference example 13 was subjected to amination in the same manner as in Example 4, and then, reacted with a corresponding isocyanate in the same manner as in Example 1, and,'if necessary, subjected to acetylation according to the conventional manner to give the compound shown in Table 22.

**Table 22**

| | | | |
|---|---|---|---|
| 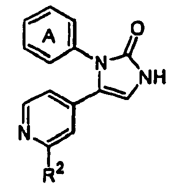 | | | |

| Example | Ring A | R² | MS ([M+H]⁺) |
|---|---|---|---|
| 192 | Phenyl | Isopropylamino | 295 |

### Example 202 (a reference Example)

The compound of Example 182 was reacted with a corresponding halide in the same manner as in Reference example 1(6) to subject to alkylation to give the compound shown in Table 23,

**Table 23**

| | | | |
|---|---|---|---|
| 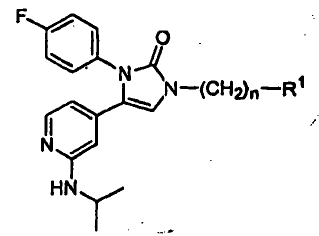 | | | |

| Example | n | R¹ | MS ([M+H]⁺) |
|---|---|---|---|
| 202* | 0 | 2-Methoxyethyl | 371 |

| | | | |
|---|---|---|---|
| *:Monohydrochloride | | | |

### Example 229 (a reference Example)

The compound of Reference example 1(5) was reacted with a corresponding compound in the same manner as in Reference example 1 (6), subsequently the resulting compound was treated in the same manner as in Examples 5 and 13 to give the compound shown in Table 26. Incidentally, the compound of Example 229 was synthesized by using 2,4-dimethoxybenzyl in place of 4-methoxybenzyl, and deprotecting with conc. hydrochloric acid/THF (70°C).

**Table 26**

| | | | |
|---|---|---|---|
| 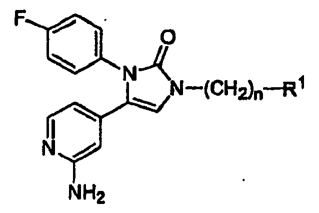 | | | |

| Example | n | R¹ | MS ([M+H]⁺) |
|---|---|---|---|
| 229 | 0 | Isopropyl | 313 |

| | | | |
|---|---|---|---|
| *:Monohydrochloride | | | |

### Example 239 (a reference Example)

The compound of Example 229 was reacted with an acid halide in the same manner as in Example 14, to give the compound shown in Table 27.

**Table 27**

| | | | | |
|---|---|---|---|---|
| 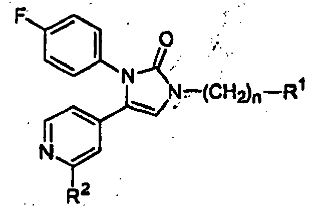 | | | | |

| Example | n | R¹ | R² | MS ([M+H]⁺) |
|---|---|---|---|---|
| 239* | 0 | Isopropyl | Cyclopropylcarbonylamino | 381 |

| | | | | |
|---|---|---|---|---|
| * :Monohydrochloride | | | | |

### Examples 296 and 300 (reference Examples)

The compound of Reference example 5(4) was reacted in the same manner as in Example 2 or Reference example 1 (6), oxidized with 3-chloroperoxybenzoic acid in the same manner as in Reference example 6(2), subsequently reacted with a corresponding amine in the same manner as in Example 17, and further, if necessary, t-butoxycarbonyl or methoxymethyl is removed in the same manner as in Example 80 or 81 to give the compounds shown in Table 33.

**Table 33**

| | | | | |
|---|---|---|---|---|
| 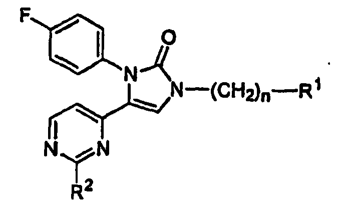 | | | | |

| Example | n | R¹ | R² | MS ([M+H]⁺) |
|---|---|---|---|---|
| 300* | 0 | Ethyl | trans-4-Hydroxycyclohexylamino | 398 |

| | | | | |
|---|---|---|---|---|
| *: Monohydrochloride ; **:Dihydrochloride | | | | |

### Example 378 (a reference Example)

The compound of Reference example 5 (4) was reacted in the same manner as in Example 2 or Reference example 1 (6), oxidized with 3-chloroperoxybenzoic acid in the same manner as in Reference example 6 (2), subsequently reacted with a corresponding amine in the same manner as in Example 17, and if necessary, t-butoxycarbonyl was removed in the same manner as in Example 80 to give the compound shown in Table 40.

**Table 40**

| | | | |
|---|---|---|---|
| 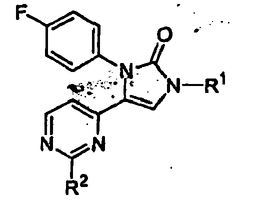 | | | |

| Example | R¹ | R² | MS ([M+H]⁺) |
|---|---|---|---|
| 378** | Isopropyl | 4-Piperidyl | 397 |

| | | | |
|---|---|---|---|
| **: Dihydrochloride | | | |

### Examples 399 to 416

The compound of Reference example 5(4) and the corresponding starting materials were reacted in the same manner as in Reference example 1(6), oxidized with 3-chloroperbenzoic acid in the same manner as in Reference example 6 (2), subsequently reacted with the corresponding amine in the same manner as in Example 17, and, if necessary, subjected to removal of t-butoxycarbonyl in the same manner as in Example 80 to give the compounds in Tables 45 and 46.

**Table 45**

| | | | |
|---|---|---|---|
| 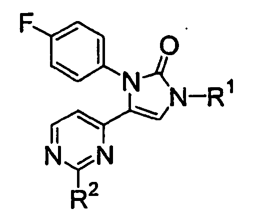 | | | |

| Example | R¹ | R² | MS ([M+H]⁺) |
|---|---|---|---|
| 399* | Ethyl | 1,1-Dioxotetrahydrothiophen-3-ylamino | 418 |
| 400* | Ethyl | trans-4-(Methylcarbamoyl)cyclohexylamino | 439 |
| 401* | Ethyl | 1,5-Dimethyl-5-hydroxyhexylamino | 428 |
| 402* | Isopropyl | 1,5-Dimethyl-5-hydroxyhexylamino | 442 |
| 403* | Ethyl | cis-4-Hydroxy-4-methylcyclohexylamino | 412 |
| 404* | Isopropyl | trans-4-Hydroxy-4-methylcyclohexylamino | 426 |
| 405* | Isopropyl | trans-4-(1-Hydroxy-1-methylethyl)cyclohexylamino | 454 |
| 406* | Ethyl | trans-4-Hydroxy-4-methylcyclohexylamino | 412 |
| 407* | Isopropyl | cis-4-Hydroxy-4-methylcyclohexylamino | 426 |

| | | | |
|---|---|---|---|
| *:monohydrochloride | | | |

**Table 46**

| | | | |
|---|---|---|---|
| 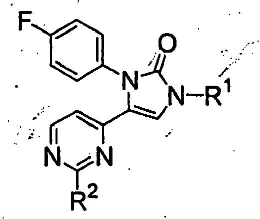 | | | |

| Example | R¹ | R² | MS ([M+H]⁺) |
|---|---|---|---|
| 408* | Ethyl | trans-4-(1-Hydroxy-1-methylethyl) cyclohexylamino | 440 |
| 409* | Ethyl | (S)-1,2-Dimethyl-2-hydroxypropylamino | 386 |
| 410* | Isopropyl | (S)-1,2-Dimethyl-2-hydroxypropylamino | 400 |
| 411* | Ethyl | 1,3-Dimethyl-3-hydroxybutylamino | 400 |
| 412* | Isopropyl | 1,3-Dimethyl-3-hydroxybutylamino | 414 |
| 413* | Isopropyl | 2-Mercapto-2-methylpropylamino | 402 |
| 414* | Isopropyl | 1,1-Bishydroxymethylpropylamino | 416 |
| 415* | Isopropyl | 2-Hydroxy-2-methylpropylamino | 386 |
| 416** | Ethyl | 4-Piperidylamino | 383 |

| | | | |
|---|---|---|---|
| *:monohydrochloride; **:dihydrochloride | | | |

### Examples 417 to 433

The compound of Reference example 5 (4) was reacted in the same manner as in Example 2 or Reference example 1 (6), reacted with methymagnesium bromide if necessary, subsequently oxidized with 3-chloroperbenzoic acid in the same manner as in Reference example 6(2), and reacted with the corresponding amine in the same manner as in Example 17 to give the compounds in Tables 47 and 48.

**Table 47**

| | | | |
|---|---|---|---|
| 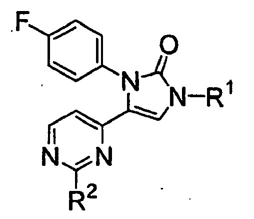 | | | |

| Example | R¹ | R² | MS ([M+H]⁺) |
|---|---|---|---|
| 417* | 1,2-Dimethyl-2-hydroxypropyl | Isopropylamino | 400 |
| 418* | 1,2-Dimethyl-2-hydroxypropyl | cis-4-Hydroxy-4-methylcyclohexylamino | 470 |
| 419* | 1,2-Dimethyl-2-hydroxypropyl | (S)-1,2-Dimethyl-2-hydroxypropylamino | 444 |
| 420* | 1,2-Dimethyl-2-hydroxypropyl | Trans-4-Hydroxy-4-methylcyclohexylamino | 470 |
| 421* | 2-Hydroxy-1,1,2-trimethylpropyl | Isopropylamino | 414 |
| 422* | 2-Hydroxy-1,1,2-trimethylpropyl | Trans-4-Hydroxy-4-methylcyclohexylamino | 484 |
| 423* | 3-Hydroxy-3-methylbutyl | Isopropylamino | 400 |
| 424* | 3-Hydroxy-3-methylbutyl | 1,1-Dimethyl-2-hydroxyethylamino | 430 |

| | | | |
|---|---|---|---|
| *:monohydrochloride | | | |

**Table 48**

| | | | |
|---|---|---|---|
| 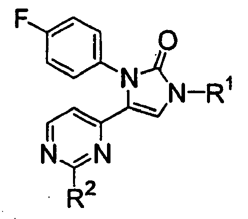 | | | |

| Example | R¹ | R² | MS ([M+H]⁺) |
|---|---|---|---|
| 425* | 3-Hydroxy-3-methylbutyl | 2,2-Dimethyl-3-hydroxypropylamino | 444 |
| 426* | 3-Hydroxy-3-methylbutyl | 1-Hydroxymethylcyclopentylamino | 456 |
| 427* | 3-Hydroxy-3-methylbutyl | trans-4-Hydroxy-4-methylcyclohexylamino | 470 |
| 428* | 3-Hydroxy-3-methylbutyl | 4-Tetrahydropyranylamino | 442 |
| 429* | 3-Hydroxy-3-methylbutyl | (R)-1,2-Dimethyl-2-hydroxypropylamino | 444 |
| 430* | 3-Hydroxy-3-methylbutyl | (S)-1,2-Dimethyl-2-hydroxypropylamino | 444 |
| 431* | 3-Hydroxy-3-methylbutyl | trans-4-Hydroxycyclohexylamino | 456 |
| 432* | 3-Hydroxy-3-methylbutyl | 1-Methanesulfonylpiperidin-4-ylamino | 519 |
| 433* | 3-Hydroxy-3-methylbutyl | 1-Ethanesulfonylpiperidin-4-ylamino | 533 |

| | | | |
|---|---|---|---|
| *:monohydrochloride | | | |

### Examples 434 to 460

The compound of Reference example 5(3) and the corresponding starting materials were reacted in the same manner as in Reference example 9, oxidized with 3-chloroperbenzoic acid in the same manner as in Reference example 6(2), and subsequently reacted with the corresponding compound in the same manner as in Example 17 to give the compounds in Tables 49 to 51.

**Table 49**

| | | | |
|---|---|---|---|
| 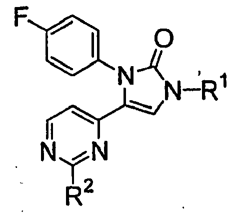 | | | |

| Example | R¹ | R² | MS ([M+H]⁺) |
|---|---|---|---|
| 434* | 4-Tetrahydropyranyl | Isobutylamino | 412 |
| 435* | 4-Tetrahydropyranyl | Isopropylamino | 398 |
| 436* | 4-Tetrahydropyranyl | trans-4-Hydroxy-4-methylcyclohexylamino | 468 |
| 437* | 4-Tetrahydropyranyl | Cyclopropylamino | 396 |
| 438* | 4-Tetrahydropyranyl | 2,2-Dimethylpropylamino | 426 |
| 439* | 1-Acetylpiperidin-4-yl | trans-4-Hydroxy-4-methylcyclohexylamino | 509 |
| 440* | 1-Acetylpiperidin-4-yl | 2,2-Dimethylpropylamino | 467 |
| 441* | 1-Acetylpiperidin-4-yl | Isopropylamino | 439 |
| 442* | 1-Acetylpiperidin-4-yl | Isobutylamino | 453 |
| 443* | 1-Acetylpiperidin-4-yl | Cyclopropylamino | 437 |
| 444* | 4-Tetrahydropyranyl | (R)-1,2-Dimethyl-2-hydroxypropylamino | 442 |
| 445* | 4-Tetrahydropyranyl | (S)-1,2-Dimethyl-2-hydroxypropylamino | 442 |
| 446* | 4-Tetrahydropyranyl | (S)-2-Hydroxy-1-methylethylamino | 414 |

| | | | |
|---|---|---|---|
| *:monohydrochloride | | | |

**Table 50**

| | | | |
|---|---|---|---|
| 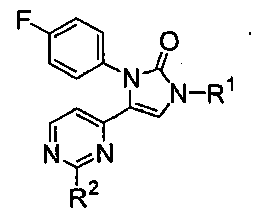 | | | |

| Example | R¹ | R² | MS ([M+H]⁺) |
|---|---|---|---|
| 447* | 4-Tetrahydropyra nyl | (S)-1-Hydroxymethylpropylamino | 428 |
| 448* | 4-Tetrahydropyra nyl | 1,1-Dimethyl-2-hydroxyethylamino | 428 |
| 449* | 4-Tetrahydropyra nyl 1-Acetylpiperidi | 4-Tetrahydropyranylamino 1,1-Dimethyl-2-hydrox | 440 |
| 450* | n-4-yl 1-Acetylpiperidi | yethylamino 4-Tetrahydropyranylam | 469 |
| 451* | n-4-yl | ino (S)-1-Hydroxymethylpr | 481 |
| 452* | 1-Acetylpiperidi n-4-yl | opylamino (S)-1,2-Dimethyl-2-hy | 469 |
| 453* | 1-Acetylpiperidi n-4-yl | droxypropylamino | 483 |

| | | | |
|---|---|---|---|
| *:monohydrochloride | | | |

**Table 51**

| | | | |
|---|---|---|---|
| 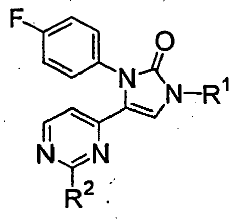 | | | |

| Example | R¹ | R² | MS ([M+H]⁺) |
|---|---|---|---|
| 454* | 4-Tetrahydropyranyl | trans-4-Hydroxycyclohexylamino | 454 |
| 455* | 1-Acetylpiperidin-4-yl | trans-4-Hydroxycyclohexylamino | 495 |
| 456* | 1-Methanesulfonylpiperidin-4-yl | trans-4-Hydroxycyclohexylamino | 531 |
| 457* | 1-Methanesulfonylpiperidin-4-yl | trans-4-Hydroxy-4-methylcyclohexylamino | 545 |
| 458* | 1-Methanesulfonylpiperidin-4-yl | Isopropylamino | 475 |
| 459* | 4-Tetrahydropyranyl | 1-Methanesulfonylpiperidin-4-ylamino | 517 |
| 460* | 1-Acetylpiperidin-4-yl | 1-Methanesulfonylpiperidin-4-ylamino | 558 |

| | | | |
|---|---|---|---|
| *:monohydrochloride | | | |

### Examples 461 to 476

The compound of Example 378 or Example 416 was subjected to alkylsulfonylation and acylation by the conventional manner, or reacted with isocyanate to give the compounds in Tables 52 and 53.

**Table 52**

| | | | |
|---|---|---|---|
| 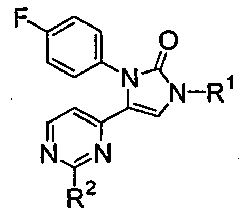 | | | |

| Example | R¹ | R² | MS ([M+H]⁺) |
|---|---|---|---|
| 461* | Isopropyl | 1-Acetylpiperidin-4-ylamino | 439 |
| 462* | Isopropyl | 1-Methanesulfonylpiperidin-4-ylamino | 475 |
| 463* | Isopropyl | 1-(Isopropylsulfonyl)piperidin-4-ylamino | 503 |
| 464* | Isopropyl | 1-(Propylsulfonyl)piperidin-4-ylamino | 503 |
| 465* | Isopropyl | 1-(Butylsulfonyl)piperidin-4-ylamino | 517 |
| 466* | Isopropyl | 1-(Isobutyloxycarbonyl)piperidin-4-ylamino | 497 |
| 467* | Isopropyl | 1-butyrylpiperidin-4-ylamino | 467 |
| 468* | Ethyl | 1-Acetylpiperidin-4-ylamino | 425 |

| | | | |
|---|---|---|---|
| *:monohydrochloride | | | |

**Table 53**

| | | | |
|---|---|---|---|
| 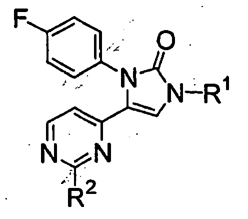 | | | |

| Example | R¹ | R² | MS ([M+H]⁺) |
|---|---|---|---|
| 469* | Ethyl | 1-Methanesulfonylpiperidin-4-ylamino | 461 |
| 470* | Ethyl | 1-Ethanesulfonylpiperidin-4-ylamino | 475 |
| 471* | Isopropyl | 1-Ethylcarbamoylpiperidin-4-ylamino | 468 |
| 472* | Isopropyl | 1-Propylcarbamoylpiperidin-4-ylamino | 482 |
| 473* | Isopropyl | 1-Isopropylcarbamoylpiperidin-4-ylamino | 482 |
| 474* | Isopropyl | 1-Ethanesulfonylpiperidin-4-ylamino | 489 |
| 475* | Isopropyl | 1-Methoxycarbonylpiperidin-4-ylamino | 455 |
| 476* | Isopropyl | 1-Ethoxycarbonylpiperidin-4-ylamino | 469 |

| | | | |
|---|---|---|---|
| *:monohydrochloride | | | |

### Examples 477, 478

The compound of Example 147 was subjected to alkylsulfonylation by the conventional manner to give the compounds in Table 54.

**Table 54**

| | | | |
|---|---|---|---|
| 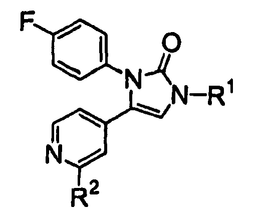 | | | |

| Example | R¹ | R² | MS ([M+H]⁺) |
|---|---|---|---|
| 477* | 1-Methanesulfonylpiperidin-4-yl | Isopropylamino | 474 |
| 478* | 1-Ethanesulfonylpiperidin-4-yl | Isopropylamino | 488 |

| | | | |
|---|---|---|---|
| *:monohydrochloride | | | |

### Reference example 1

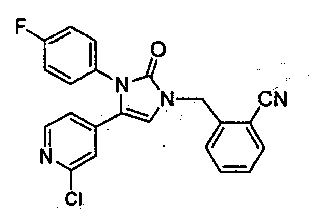

(1) In 440 ml of THF was suspended 22 g of 2-chloroisonicotinic acid, and under nitrogen flow, the mixture was cooled to -70 °C or lower, 245 ml of methyl lithium (1.14 M solution in diethyl ether) was added dropwise to the mixture. After stirring at the same temperature for an hour, a temperature of the mixture was raised to 0°C over an hour, and stirred at the same temperature for further an hour. To the reaction mixture was added 500 ml of water, and the reaction mixture was extracted with ethyl acetate, washed with brine and dried over magnesium sulfate. Activated charcoal was added to the mixture, and after filtration, the filtrate was concentrated under reduced pressure to give 19.5 g of 4-acetyl-2-chloropyridine as colorless crystals. Melting point: 36°C.
(2) In 550 ml of ethanol were suspended 55.1 g of the compound obtained in (1), 49.2 g of hydroxylamine hydrochloride and 58.1 g of sodium acetate, and the mixture was refluxed under heating for an hour. After cooling the mixture to room temperature by allowing to stand, ethanol was distilled away under reduced pressure and precipitated crystals were collected by filtration and washed with water. The crystals were air-dried at 60°C overnight to give 55 g of 1-(2-chloropyridin-4-yl)ethanone oxime as colorless crystals. Melting point: 143°C.
(3) In methylene chloride were suspended 105 g of the compound obtained in (2) and 123 g of tosyl chloride, and under ice-cooling, 94 ml of triethylamine was added dropwise to the mixture, and the mixture was raised to room temperature and stirred for 4 hours. To the reaction mixture was added 500 ml of water, and the mixture was extracted with methylene chloride, washed with brine and dried over magnesium sulfate. After filtration, the mixture was concentrated under reduced pressure, and the resulting crystals were collected by filtration and washed with isopropyl ether to give 192 g of 1-(2-chloropyridin-4-yl)ethanone oxime tosylate as colorless crystals. Melting point: 153°C.
(4) Under nitrogen flow, 3.11 g of sodium metal was added to 220 ml of anhydrous ethanol at room temperature, and the mixture was dissolved under stirring. The solution was ice-cooled, and 40 g of the compound obtained in (3) was added thereto, then the mixture was stirred at room temperature for an hour. To the mixture was added 220 ml of anhydrous ether, and insoluble matters were removed. To the filtrate was added 62 ml of 4N hydrochloric acid/dioxane solution under ice-cooling and the mixture was stirred for 15 minutes. After the reaction mixture was concentrated under reduced pressure, the residue was dissolved in water and the solution was made alkaline by addition of potassium carbonate. This mixture was extracted with ethyl acetate several times, and the combined extracts were washed with brine and dried over magnesium sulfate. After concentration under reduced pressure, 100 ml of hexane was added to the residue and red insoluble matters were removed by filtration. The filtrate was concentrated under reduced pressure, hexane was again added to the concentrate and insoluble matters were removed by filtration through Celite. The filtrate was concentrated under reduced pressure and dried by a vacuum pump to give 26.9 g of 2-(2-chloropyridin-4-yl)-2,2-diethoxyethylamine as reddish oily product.
(5) A solution, in which 20 g of the compound obtained in (4) was dissolved in 50 ml of THF, was water-cooled, and 11.2 g of 4-fluorophenylisocyanate was added dropwise thereto. After dropwise addition, the reaction mixture was concentrated under reduced pressure, and 30 ml of conc. hydrochloric acid was added to the obtained residue and the mixture was stirred at room temperature overnight. The reaction mixture was added to ice-cooled 180 ml of 2N aqueous NaOH solution to neutralize the mixture, and after collecting the precipitated crystals by filtration, the crystals were washed with water and ether. The crystals were air-dried at 60°C to give 22.3 g of 5-(2-chloropyridin-4-yl)-1-(4-fluorophenyl)-4-imidazolin-2-one as colorless crystals. Melting point: 270°C.
(6) In 50 ml of DMF was suspended 10 g of the compound obtained in (5), and under ice-cooling, 1.46 g of 63% sodium hydride was added to the suspension, then the mixture was stirred at room' temperature for 30 minutes. The mixture was again ice-cooled, and after adding 7.44 g of 2-cyanobenzyl bromide, the mixture was stirred at room temperature for 3 hours. The reaction mixture was poured into 250 ml of ice-cold water, extracted with ethyl acetate. The extract was washed with water and brine, and dried over anhydrous magnesium sulfate. After concentration under reduced pressure, the residue was purified by silica gel column chromatography to give 11.4 g of 4-(2-chloropyridin-4-yl)-3-(4-fluorophenyl)-1-(2-cyanobenzyl)-4-imidazolin-2-one as colorless crystals. Melting point: 109°C.

### Reference example 2

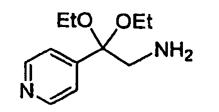

By using 4-acetylpyridine (commercially available product) as a starting material, the same treatments as in Reference examples 1(2) to (4) were carried out to give 2,2-diethoxy-2-pyridin-4-yl ethylamine as brownish oily product.

### Reference example 3

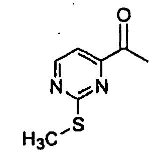

(1) A mixture of 100 g of 3,3-dimethoxy-2-butanone and 99.2 g of N,N-dimethylformamide dimethylacetal was stirred at 100°C for 42 hours. After cooling the reaction mixture, the mixture was concentrated under reduced pressure to give 141 g of 1-dimethylamino-4,4-dimethoxy-1-penten-3-one.
(2) In 800 ml of methanol was dissolved 141 g of the compound obtained in (1), and after adding 114 g of thiourea and 292 g of 28% sodium methoxide-methanol, the mixture was stirred at 70°C for 3 hours. The mixture was ice-cooled, and after adding 215 g of methyl iodide drowise, the mixture was stirred at room temperature for an hour. After concentration of the reaction mixture, water was added to the mixture and the resulting mixture was extracted with ethyl acetate. The organic layer was washed, dried and concentrated to give 142 g of 4-(1,1-dimethoxyethyl)-2-methylsulfanylpyrimidine.
(3) In 570 ml of acetone was dissolved 142 g of the compound obtained in (2), and under ice-cooling, 114 ml of 6M hydrochloric acid was added to the solution and the mixture was stirred at room temperature for 3 hours. After adding 450 ml of water to the mixture, the solvent was removed and the residue was extracted with ethyl acetate. The organic layer was washed, dried and concentrated to give 107 g of 1-(2-methylsulfanylpyrimidin-4-yl)ethanone.

### Reference example 4

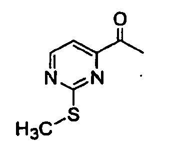

(1) A mixture comprising 16.4 g of 4-chloro-2-methylsulfanylpyrimidine, 38 g of tributyl (1-ethoxyvinyl) tin, 1.43 g of bis(triphenylphosphine)palladium (II) dichloride and 100 ml of DMF was stirred at 80°C for 3 hours. After cooling the reaction mixture, 300 ml of ethyl acetate and 17.8 g of potassium fluoride were added to the mixture, and the resulting mixture was stirred at room temperature overnight. After filtration with Celite, the filtrate was washed, dried and concentrated. The residue was purified by silica gel column chromatography (hexane:ethylacetate=20:1) to give 18.9 g of 4-(1-ethoxyvinyl)-2-methylsulfanylpyrimidine.
(2) In 200 ml of acetone was dissolved 18.9 g of the compound obtained in (1), 60 ml of 4M hydrochloric acid was added to the solution and the mixture was stirred at room temperature for an hour. The reaction mixture was added to a saturated aqueous sodium bicarbonate solution, and extracted with ethyl acetate. The organic layer was washed, dried and concentrated to give 15.9 g of 1-(2-methylsulfanylpyrimidin-4-yl)ethanone.

### Reference example 5

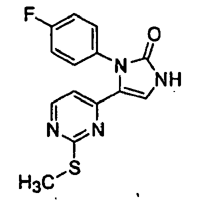

(1) In 180 ml of methanol was dissolved 17.6 g of the compound obtained in Reference example 3 (3) or Reference example 4 (2), 14.5 g of hydroxylamine hydrochloride and 17.2 g of sodium acetate were added to the solution, and the mixture was refluxed under heating for 30 minutes. After cooling the reaction mixture, the solvent was removed, water was added to the residue and the mixture was extracted with ethyl acetate. The organic layer was washed, dried and concentrated. To the residue was added hexane and the precipitated crystals were collected by filtration to give 18.3 g of 1-(2-methylsulfanylpyrimidin-4-yl)ethanone oxime. Melting point: 150-152°C.
(2) In 1200 ml of methylene chloride was suspended 89 g of the compound obtained in (1), and 81.2 ml of triethylamine and 102 g of tosyl chloride were added to the suspension, and the mixture was stirred at room temperature overnight. The reaction mixture was washed, dried and concentrated. To the residue was added diethyl ether and the precipitated crystals were collected by filtration to give 159 g of 1-(2-methylsulfanylpyrimidin-4- yl)ethanoneoxime tosylate. Melting point: 141-142°C.
(3) To 30 ml of methanol solution containing 12.9 g of 28% sodium methoxide-methanol was added dropwise 120 ml of a THF solution containing 15 g of the compound obtained in (2) under ice-cooling, and the mixture was stirred at room temperature overnight. To the mixture was added 100 ml of 4M hydrochloric acid-dioxane solution under ice-cooling, and after stirring at room temperature for 4 hours, the reaction mixture was concentrated. The residue was added to an aqueous potassium carbonate solution and extracted with chloroform. The organic layer was dried and concentrated, and the residue was purified by silica gel column chromatography (chloroform:methanol= 15:1) to give 8.14 g of 2,2-dimethoxy-2-(2-methylsulfanylpyrimidin-4-yl)ethylamine.
(4) To 120 ml of a THF solution containing 8 g of the compound obtained in (3) was added dropwise under ice-cooling 30 ml of a THF solution containing 4.78 g of 4-fluorophenyl isocyanate, and the mixture was stirred at room temperature for 30 minutes. After 120 ml of conc. hydrochloric acid was added to the mixture under ice-cooling, the resulting mixture was stirred at room temperature overnight. Precipitated crystals were collected by filtration, washed with water and ether, and dried to give 7.35 g of 1-(4-fluorophenyl)-5-(2-methylsulfanylpyrimidin-4-yl)-4-imidazolin-2-one. Melting point: 260-261°C.

### Reference example 6

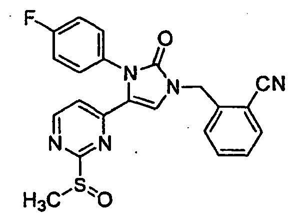

(1) To 40 ml of a DMF solution containing 2.6 g of the compound obtained in Reference example 5(4) was added 327 mg of sodium hydride at room temperature, and the mixture was stirred at room temperature for 30 minutes. To the mixture was added 1.77 g of 2-cyanobenzyl bromide, and after stirring at room temperature for 30 minutes, 33 mg of sodium hydride and 85 mg of 2-cyanobenzyl bromide were added to the mixture, and the resulting mixture was stirred at room temperature for an hour. Water was added to the reaction mixture and the mixture was extracted with ethyl acetate. The organic layer was washed, dried and concentrated, and crystallized from diethyl ether to give 3.28 g of 1-(2-cyanobenzyl)-3-(4-fluorophenyl)-4-(2-methylsulfanylpyrimidin-4-yl)-4-imidazolin-2-one. Melting point: 141-142°C.
(2) To a chloroform solution containing 3.27 g of the compound obtained in (1) was added 2.03 g of 3-chloroperoxybenzoic acid at room temperature, and the mixture was stirred at room temperature for an hour. To the reaction mixture was added 1.16 g of calcium hydroxide and the mixture was stirred at room temperature for 2 hours, and then, filtered through Celite, and the filtrate was concentrated. The residue was crystallized from ethyl acetate to give 2.39 g of 1-(2-cyanobenzyl)-3-(4-fluorophenyl)-4-(2-methylsulfinylpyrimidin-4-yl)-4-imidazolin-2-one. Melting point: 133-136°C.

### Reference example 7

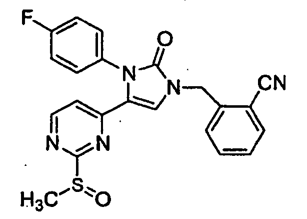

(1) To 150 ml of a methanol solution containing 1.47 g of the compound obtained in Reference example 5 (4) was added dropwise 10 ml of an aqueous solution containing 1.79 g of Oxone® at room temperature. After 30 minutes and 2 hours, 2 ml of an aqueous solution containing 299 mg of Oxon® was added dropwise, and the mixture was stirred at room temperature for 2 hours. After removing insoluble matters by filtration, the filtrate was concentrated, an aqueous sodium bicarbonate solution was added to the concentrate and the mixture was extracted with chloroform. The organic layer was washed, dried and concentrated, and the precipitated crystals were collected by a mixed solvent of ethyl, acetate-ether (1:1) to give 1. 03 g of 1-(4-fluorophenyl)-5-(2-methylsulfinylpyrimidin-4-yl)-4-imidazolin-2-one. Melting point: 208-211°C (decomposed).
(2) The compound (930 mg) obtained in (1) was,treated in the same manner as in the above-mentioned Reference example 6(1) to give 541 mg of 1-(2-cyanobenzyl)-3-(4-fluorophenyl)-4-(2-methylsulfinylpyrimidin-4-yl)-4-imidazolin-2-one.

### Reference example 8

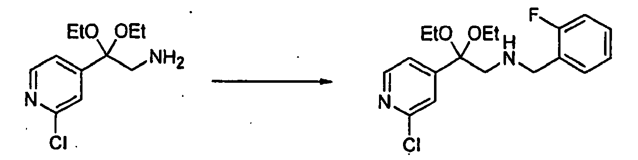

In 10 ml of methanol was dissolved 1. 0 g of the compound obtained in Reference example 1 (4), 0. 51 g of 2-fluorobenzaldehyde was added to the solution, and the mixture was stirred at room temperature for 30 minutes. To the mixture was added 155 mg of sodium borohydride, and the resulting mixture was further stirred at room temperature for an hour. After concentration under reduced pressure, water was added to the reside and the mixture was extracted with ethyl acetate. The extract was washed with brine and dried over anhydrous magnesium sulfate. After concentration under reduced pressure, the residue was purified by silica gel column chromatography (hexane:ethylacetate=2:1) to give 1.45 g the title compound as an oily product.

### Reference example 9

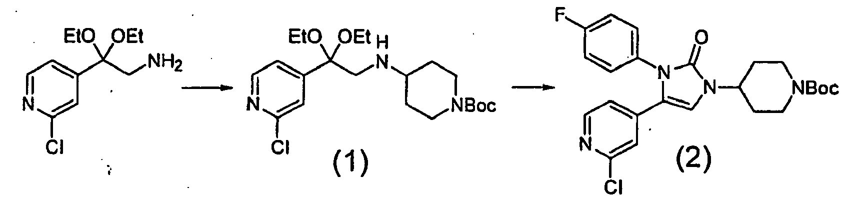

The compound (5 g) obtained in Reference example 1(4) and a corresopnding starting material were treated in the same manner as in Reference example 8 to give 8.47 g of Compound (1) . Compound (1) (3 g) was treated in the same manner as in Example 1 to carry out cyclization, subsequently the resulting compound was dissolved in 20 ml of THF, 1.1 g of Boc₂O was added thereto. The resulting mixture was stirred at room temperature for 30 minutes, concentrated under reduced pressure and diisopropyl ether was added to the residue, and the residue was collected by filtration to give 2.53 g of Compound (2).

### Reference example 10

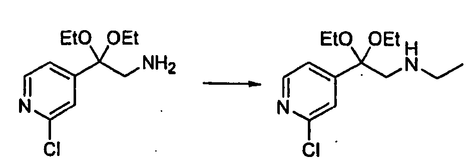

A mixture comprising 3.8 g of the compound obtained in Reference example 1 (4), 1. 7 ml of ethyl iodide and 3.0 ml of triethylamine was stirred at 50°C overnight. After neutralizing with 2N aqueous NaOH solution, the reaction mixture was extracted with chloroform and dried over anhydrous magnesium sulfate. The resulting mixture was purified by NH silica gel column chromatography (hexane: ethyl acetate=4:1) to give 1. 9 g of the title compound as an oily product.

### Reference example 11

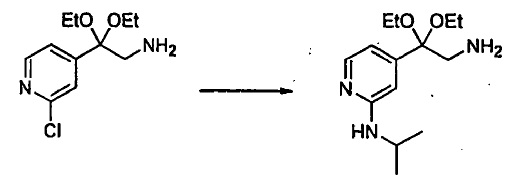

In 75 ml of toluene were suspended 5. 0 g of the compound obtained in Reference example 1(4), 35 ml of isopropylamine, 458 mg of palladium acetate, 1.28 g of 2,2'-bis(diphenylphosphino)-1,1'-binaphthyl and 3.0 g of sodium t-butoxide, and under nitrogen flow, the mixture was stirred under heating at 70°C for 8 hours. After concentration under reduced pressure, water was added to the residue, and the mixture was extracted with chloroform, washed with brine and dried over anhydrous magnesium sulfate. After concentration under reduced pressure, the residue was purified by silica gel column chromatography (chloroform:methanol= 10:1) to give 4.3 g of the title compound as an oily product.

### Reference example 12

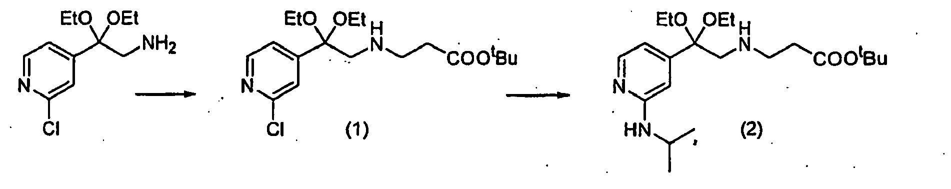

A mixture comprising 2.0 g of the compound obtained in Reference example 1(4), 0.82 ml of t-butyl acrylate and 10 ml of THF was stirred under reflux for 4 days. The reaction mixture was concentrated under reduced pressure to give 3.1 g of Compound (1) as an oily product. Then, Compound (1) and a corresponding starting material were treated in the same manner as in Example 4 to give 2.12 g of Compound (2) as an oily product.

### Reference example 13

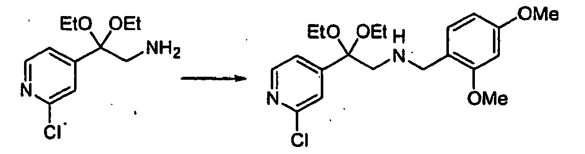

The compound (5.0 g) obtained in Reference example 1(4) was reacted with 2,4-dimethoxybenzaldehyde in the same manner as in Reference example 8 to give 6.4 g of the title compound.

### Reference example 14

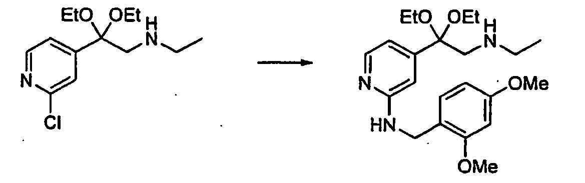

The compound (1.39g) of Reference example 10 was reacted with 2,4-dimethoxybenzylamine in the same manner as in Reference example 11 to give 1.58 g of the title compound.

### Reference example 15

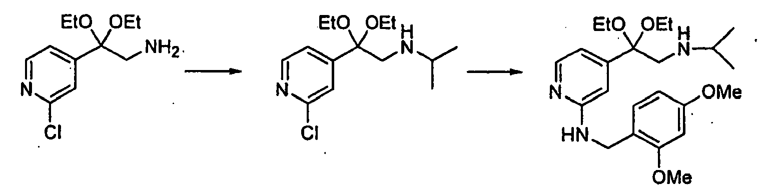

The compound (10.0 g) of Reference example 1 (4) was reacted with a corresponding starting material in the same manner as in Reference example 8, and then, reacted with 2,4-dimethoxybenzylamine in the same manner as in Reference example 11 to give 9.75 g of the title compound.

### Reference example 16

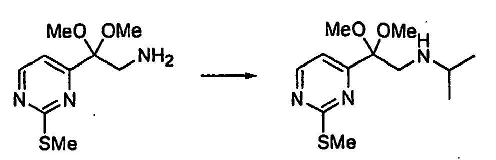

The compound (26.8 g) of Reference example 5(3) and a corresponding starting material were treated in the same manner as in Reference example 8 to give 30.8 g of the title compound.

### Reference example 17

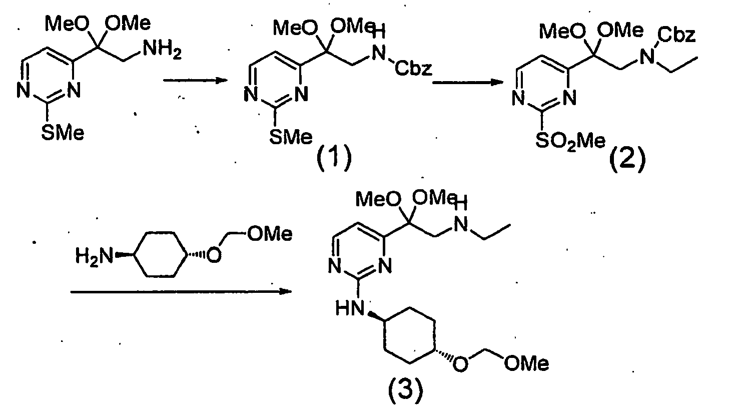

(1) In 30 ml of methylene chloride was dissolved 3.0 g of the compound of Reference example 5(3), 3.65 ml of triethylamine was added to the solution, and under ice-cooling, 3.35 g of benzyloxycarbonyl chloride was added dropwise to the mixture, and the mixture was stirred at room temperature overnight. The reaction mixture was washed with water and brine, and dried over anhydrous magnesium sulfate. After concentration under reduced pressure, the residue was purified by silica gel column chromatography to give 2.23 g of Compound (1) as colorless crystals.
   MS 364 ([M+H]⁺)
(2) In 17 ml of DMF was dissolved 4.2 g of Compound (1), and under ice-cooling, 528 mg of sodium hydride was added to the solution, and the mixture was stirred at room temperature for an hour. The mixture was again ice-cooled, 1.39 ml of ethyl iodide was added thereto, and the resulting mixture was stirred at room temperature for 30 minutes. Water was added to the reaction mixture and the mixture was extracted with ethyl acetate, the extract was washed with water and brine, and dried over anhydrous magnesium sulfate. After concentration under reduced pressure, the residue was dissolved in 50 ml of chloroform, 6.26 g of 3-chloroperoxybenzoic acid was added to the mixture at room temperature, and the resulting mixture was stirred at the same temperature for 30 minutes. To the reaction mixture was added 2.58 g of calcium hydroxide and after stirring the mixture, the insoluble matters were removed by filtration. The filtrate was concentrated under reduced pressure, and purified by silica gel column chromatography to give 4.55 g of Compound (2) as a colorless oily product.
   MS 423 ([M+H]⁺)
(3) In 30 ml of dioxane was dissolved 2.19 g of Compound (2), 1.65 g of trans-4-(Methoxymethoxy) cyclohexylamine and 1.08 ml of 1,1'-diisopropylethylamine were added to the solution, and the mixture was stirred at 100 °C for 14 hours. Water was added to the reaction mixture and the mixture was extracted with ethyl acetate, washed with brine and dried over anhydrous magnesium sulfate. After concentration under reduced pressure, the residue was purified by silica gel column chromatography to give 2.0 g of a brownish oily product. This product was dissolved in 40 ml of methanol, 1 g of 10% palladium-carbon was added thereto, and the mixture was subjected to catalytic reduction under hydrogen pressure (2.7 atm) for 2 hours. Palladium was removed by filtration, and after concentration under reduced pressure, the residue was purified by NH silica gel column chromatography to give 1.04 g of Compound (3) as a brownish oily product.
   MS 369 ([M+H]⁺)

### Experimental Example 1 (pharmacological test)

### Inhibition of lipopolysaccharide (LPS)-stimulated TNF-α production in mice in vivo

Tests were carried out to measure an inhibitory effects of the compounds of the present invention on LPS-stimulated TNF-α production in mice.

To Balb/cAnNCrj mice (6-8 weeks old, female, available from Japan Charlesriver, Co.) were administered test compounds (10 mg/kg, p.o.) dissolved in 0.5% methyl cellulose and 0.2% PEG-60 hydrogenated caster oil (HCO60, available from Nikko Chemicals, Co.). After 30 minutes, LPS (E. coli 0111:B4, available from Difco, with a final concentration of 1 mg/kg adjusted by phosphate buffered saline) was administered (0.4 ml/head, i.p.). 90 minutes later, blood was collected from abdominal vein of the mouse under diethyl ether anesthesia. The collected blood was subjected to centrifugation with 3000g to collect serum. TNF-α in the sera was measured by DuoSet mouse TNF-α ELISA kit (trade name, available from genzymeTECHNE).

As a result, the compounds of the present invention significantly reduced the production of TNF-α as shown in Table 78.

**Table 78**

| Examples | TNF-α inhibition rate |
|---|---|
| 182 | 64% |
| 202 | 57% |
| 239 | 69% |
| 296 | 52% |
| 300 | 57% |

### Industrial Applicability

According to the present invention, a novel 4-imidazolin-2-one compound having excellent p38MAP kinase inhibitory activity, which is useful as a medicine, can be provided.

## Claims

1. A compound selected from free base compounds of structures 399 to 478, or a pharmaceutically acceptable salt thereof.
| | | |
|---|---|---|
| 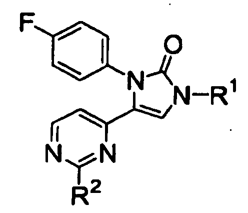 | | |
| | R¹ | R² |
|---|---|---|
| 399 | Ethyl | 1,1-Dioxotetrahydrothiophen-3-ylamino |
| 400 | Ethyl | trans-4-(Methylcarbamoyl)cyclohexylamino |
| 401 | Ethyl | 1,5-Dimethyl-5-hydroxyhexylamino |
| 402 | Isopropyl | 1,5-Dimethyl-5-hydroxyhexylamino |
| 403 | Ethyl | cis-4-Hydroxy-4-methylcyclohexylamino |
| 404 | Isopropyl | trans-4-Hydroxy-4-methylcyclohexylamino |
| 405 | Isopropyl | trans-4-(1-Hydroxy-1-methylethyl)cyclohexylamino |
| 406 | Ethyl | trans-4-Hydroxy-4-methylcyclohexylamino |
| 407 | Isopropyl | cis-4-Hydroxy-4-methylcyclohexylamino |
| 408 | Ethyl | trans-4-(1-Hydroxy-1-methylethyl)cyclohexylamino |
| 409 | Ethyl | (S)-1,2-Dimethyl-2-hydroxypropylamino |
| 410 | Isopropyl | (S)-1,2-Dimethyl-2-hydroxypropylamino |
| 411 | Ethyl | 1,3-Dimethyl-3-hydroxybutylamino |
| 412 | Isopropyl | 1,3-Dimethyl-3-hydroxybutylamino |
| 413 | Isopropyl | 2-Mercapto-2-methylpropylamino |
| 414 | Isopropyl | 1,1-Bishydroxymethylpropylamino |
| 415 | Isopropyl | 2-Hydroxy-2-methylpropylamino |
| 416 | Ethyl | 4-Piperidylamino |
| 417 | 1,2-Dimethyl-2-hydroxypropyl | Isopropylamino |
| 418 | 1,2-Dimethyl-2-hydroxypropyl | cis-4-Hydroxy-4-methylcyclohexylamino |
| 419 | 1,2-Dimethyl-2-hydroxypropyl | (S)-1,2-Dimethyl-2-hydroxypropylamino |
| 420 | 1,2-Dimethyl-2-hydroxypropyl | Trans-4-Hydroxy-4-methylcyclohexylamino |
| 421 | 2-Hydroxy-1,1,2-trimethylpropyl | Isopropylamino |
| 422 | 2-Hydroxy-1,1,2-trimethylpropyl | Trans-4-Hydroxy-4-methylcyclohexylamino |
| 423 | 3-Hydroxy-3-methylbutyl | Isopropylamino |
| 424 | 3-Hydroxy-3-methylbutyl | 1,1-Dimethyl-2-hydroxyethylamino |
| 425 | 3-Hydroxy-3-methylbutyl | 2,2-Dimethyl-3-hydroxypropylamino |
| 426 | 3-Hydroxy-3-methylbutyl | 1-Hydroxymethylcyclopentylamino |
| 427 | 3-Hydroxy-3-methylbutyl | trans-4-Hydroxy-4-methylcyclohexylamino |
| 428 | 3-Hydroxy-3-methylbutyl | 4-Tetrahydropyranylamino |
| 429 | 3-Hydroxy-3-methylbutyl | (R)-1,2-Dimethyl-2-hydroxypropylamino |
| 430 | 3-Hydroxy-3-methylbutyl | (S)-1,2-Dimethyl-2-hydroxypropylamino |
| 431 | 3-Hydroxy-3-methylbutyl | trans-4-Hydroxycyclohexylamino |
| 432 | 3-Hydroxy-3-methylbutyl | 1-Methanesulfonylpiperidin-4-ylamino |
| 433 | 3-Hydroxy-3-methylbutyl | 1-Ethanesulfonylpiperidin-4-ylamino |
| 434 | 4-Tetrahydropyranyl | Isobutylamino |
| 435 | 4-Tetrahydropyranyl | Isopropylamino |
| 436 | 4-Tetrahydropyranyl | trans-4-Hydroxy-4-methylcyclohexylamino |
| 437 | 4-Tetrahydropyranyl | Cyclopropylamino |
| 438 | 4-Tetrahydropyranyl | 2,2-Dimethylpropylamino |
| 439 | 1-Acetylpiperidin-4-yl | trans-4-Hydroxy-4-methylcyclohexylamino |
| 440 | 1-Acetylpiperidin-4-yl | 2,2-Dimethylpropylamino |
| 441 | 1-Acetylpiperidin-4-yl | Isopropylamino |
| 442 | 1-Acetylpiperidin-4-yl | Isobutylamino |
| 443 | 1-Acetylpiperidin-4-yl | Cyclopropylamino |
| 444 | 4-Tetrahydropyranyl | (R)-1,2-Dimethyl-2-hydroxypropylamino |
| 445 | 4-Tetrahydropyranyl | (S)-1,2-Dimethyl-2-hydroxypropylamino |
| 446 | 4-Tetrahydropyranyl | (S)-2-Hydroxy-1-methylethylamino |
| 447 | 4-Tetrahydropyranyl | (S)-1-Hydroxymethylpropylamino |
| 448 | 4-Tetrahydropyranyl | 1,1-Dimethyl-2-hydroxyethylamino |
| 449 | 4-Tetrahydropyranyl | 4-Tetrahydropyranylamino |
| 450 | 1-Acetylpiperidin-4-yl | 1,1-Dimethyl-2-hydroxyethylamino |
| 451 | 1-Acetylpiperidin-4-yl | 4-Tetrahydropyranylamino |
| 452 | 1-Acetylpiperidin-4-yl | (S)-1-Hydroxymethylpropylamino |
| 453 | 1-Acetylpiperidin-4-yl | (S)-1,2-Dimethyl-2-hydroxypropylamino |
| 454 | 4-Tetrahydropyranyl | trans-4-Hydroxycyclohexylamino |
| 455 | 1-Acetylpiperidin-4-yl | trans-4-Hydroxycyclohexylamino |
| 456 | 1-Methanesulfonylpiperidin-4-yl | trans-4-Hydroxycyclohexylamino |
| 457 | 1-Methanesulfonylpip eridin-4-yl | trans-4-Hydroxy-4-methylcy clohexylamino |
| 458 | 1-Methanesulfonylpip eridin-4-yl | Isopropylamino |
| 459 | 4-Tetrahydropyranyl | 1-Methanesulfonylpiperidin -4-ylamino |
| 460 | 1-Acetylpiperidin-4-yl | 1-Methanesulfonylpiperidin -4-ylamino |
| 461 | Isopropyl | 1-Acetylpiperidin-4-ylamino |
| 462 | Isopropyl | 1-Methanesulfonylpiperidin-4-ylamino |
| 463 | Isopropyl | 1-(Isopropylsulfonyl)piperidin-4-ylamino |
| 464 | Isopropyl | 1-(Propylsulfonyl)piperidin-4-ylamino |
| 465 | Isopropyl | 1-(Butylsulfonyl)piperidin-4-ylamino |
| 466 | Isopropyl | 1-(Isobutyloxycarbonyl)piperidin-4-ylamino |
| 467 | Isopropyl | 1-butyrylpiperidin-4-ylamino |
| 468 | Ethyl | 1-Acetylpiperidin-4-ylamino |
| 469 | Ethyl | 1-Methanesulfonylpiperidin-4-ylamino |
| 470 | Ethyl | 1-Ethanesulfonylpiperidin-4-ylamino |
| 471 | Isopropyl | 1-Ethylcarbamoylpiperidin-4-ylamino |
| 472 | Isopropyl | 1-Propylcarbamoylpiperidin-4-ylamino |
| 473 | Isopropyl | 1-Isopropylcarbamoylpiperidin-4-ylamino |
| 474 | Isopropyl | 1-Ethanesulfonylpiperidin-4-ylamino |
| 475 | Isopropyl | 1-Methoxycarbonylpiperidin-4-ylamino |
| 476 | Isopropyl | 1-Ethoxycarbonylpiperidin-4-ylamino |
| 477 | 1-Methanesulfonylpiperidin-4-yl | Isopropylamino |
| 478 | 1-Ethanesulfonylpiperidin-4 -yl | Isopropylamino |

2. A compound according to claim 1 which is a free base compound of structure 415, or a pharmaceutically acceptable salt thereof.
| | | |
|---|---|---|
| 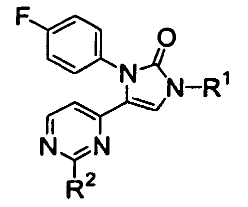 | | |
| example | R¹ | R² |
|---|---|---|
| 415 | Isopropyl | 2-Hydroxy-2-methylpropylamino |

3. A compound according to claim 1 which is a free base compound of structure 417, or a pharmaceutically acceptable salt thereof.
| | | |
|---|---|---|
| 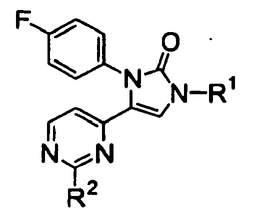 | | |
| example | R¹ | R² |
|---|---|---|
| 417 | 1,2-Dimethyl-2-hydroxypropyl | Isopropylamino |

4. A compound according to claim 1 which is a free base compound of structure 436, or a pharmaceutically acceptable salt thereof.
| | | |
|---|---|---|
| 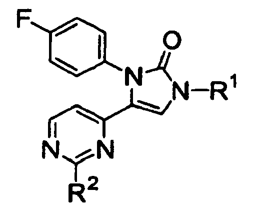 | | |
| example | R¹ | R² |
|---|---|---|
| 436 | 4-Tetrahydropyranyl | trans-4-Hydroxy-4-methyl cyclohexylamino |

5. A compound according to claim 1 which is a free base compound of structure 460, or a pharmaceutically acceptable salt thereof.
| | | |
|---|---|---|
| 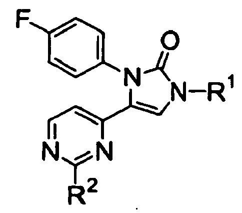 | | |
| example | R¹ | R² |
|---|---|---|
| 460 | 1-Acetylpiperidin-4-yl | 1-Methanesulfonylpiperid in-4-ylamino |

6. A pharmaceutical composition comprising the compound according to any one of Claims 1 to 5 or a pharmaceutically acceptable salt thereof, and a pharmaceutically acceptable carrier.

7. Use of the compound according to any one of Claims 1 to 5 or a pharmaceutically acceptable salt thereof for the preparation of a p38 MAP kinase inhibitor.

8. Use of the compound according to any one of Claims 1 to 5 or a pharmaceutically acceptable salt thereof for the preparation of an agent for prophylaxis or treatment for a disease selected from the group consisting of arthritis, inflammatory bowel disease, inflammatory dermal disease, inflammatory respiratory disease, inflammatory optical disease, nephritis, hepatitis, systemic inflammatory disease, shock, cerebrovascular disease, ischemic cardiac disease, osteoporosis, multiple sclerosis, diabetes, malignant tumor, cachexia, Alzheimer's disease, Parkinson's disease, acquired immunodeficiency syndrome, arterial sclerosis, disseminated intravascular coagulation syndrome, rejection and graft-versus-host diseases by organ transplantation.

9. A compound according to any one of Claims 1 to 5 or a pharmaceutically acceptable salt thereof for use in the prophylaxis or treatment of a disease selected from the group consisting of arthritis, inflammatory bowel disease, inflammatory dermal disease, inflammatory respiratory disease, inflammatory optical disease, nephritis, hepatitis, systemic inflammatory disease, shock, cerebrovascular disease, ischemic cardiac disease, osteoporosis, multiple sclerosis, diabetes, malignant tumor, cachexia, Alzheimer's disease, Parkinson's disease, acquired immunodeficiency syndrome, arterial sclerosis, disseminated intravascular coagulation syndrome, rejection and graft-versus-host diseases by organ transplantation.

## Patentansprüche

1. Verbindung, die aus den Verbindungen der freien Base der Strukturen 399 bis 478 ausgewählt ist, oder ein pharmazeutisch akzeptables Salz derselben.
| | | |
|---|---|---|
| 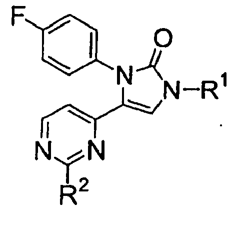 | | |
| | R¹ | R² |
|---|---|---|
| 399 | Ethyl | 1,1-Dioxotetrahydrothiophen-3-ylamino |
| 400 | Ethyl | trans-4-(Methylcarbamoyl)cyclohexylamino |
| 401 | Ethyl | 1,5-Dimethyl-5-hydroxyhexylamino |
| 402 | Isopropyl | 1,5-Dimethyl-5-hydroxyhexylamino |
| 403 | Ethyl | cis-4-Hydroxy-4-methylcyclohexylamino |
| 404 | Isopropyl | trans-4-Hydroxy-4-methylcyclohexylamino |
| 405 | Isopropyl | trans-4-(1-Hydroxy-1-methylethyl) cyclohexylamino |
| 406 | Ethyl | trans-4-Hydroxy-4-methylcyclohexylamino |
| 407 | Isopropyl | cis-4-Hydroxy-4-methylcyclohexylamino |
| 408 | Ethyl | trans-4-(1-Hydroxy-1-methylethyl)cyclohexylamino |
| 409 | Ethyl | (S)-1,2-Dimethyl-2-hydroxypropylamino |
| 410 | Isopropyl | (S)-1,2-Dimethyl-2-hydroxypropylamino |
| 411 | Ethyl | 1,3-Dimethyl-3-hydroxybutylamino |
| 412 | Isopropyl | 1,3-Dimethyl-3-hydroxybutylamino |
| 413 | Isopropyl | 2-Mercapto-2-methylpropylamino |
| 414 | Isopropyl | 1,1-Bishydroxymethylpropylamino |
| 415 | Isopropyl | 2-Hydroxy-2-methylpropylamino |
| 416 | Ethyl | 4-Piperidylamino |
| 417 | 1,2-Dimethyl-2-hydroxypropyl | Isopropylamino |
| 418 | 1,2-Dimethyl-2-hydroxypropyl | cis-4-Hydroxy-4-methylcyclohexylamino |
| 419 | 1,2-Dimethyl-2-hydroxypropyl | (S}-1,2-Dimethyl-2-hydroxypropylamino |
| 420 | 1,2-Dimethyl-2-hydroxypropyl | trans-4-Hydroxy-4-methylcyclohexylamino |
| 421 | 2-Hydroxy-1,1,2-trimethylpropyl | Isopropylamino |
| 422 | 2-Hydroxy-1,1,2-trimethylpropyl | trans-4-Hydroxy-4-methylcyclohexylamino |
| 423 | 3-Hydroxy-3-methylbutyl | Isopropylamino |
| 424 | 3-Hydroxy-3-methylbutyl | 1,1-Dimethyl-2-hydroxyethylamino |
| 425 | 3-Hydroxy-3-methylbutyl | 2,2-Dimethyl-3-hydroxypropylamino |
| 426 | 3-Hydroxy-3-methylbutyl | 1-Hydroxymethylcyclopentylamino |
| 427 | 3-Hydroxy-3-methylbutyl | trans-4-Hydroxy-4-methylcyclohexylamino |
| 428 | 3-Hydroxy-3-methylbutyl | 4-Tetrahydropyranylamino |
| 429 | 3-Hydroxy-3-methylbutyl | (R)-1,2-Dimethyl-2-hydroxypropylamino |
| 430 | 3-Hydroxy-3-methylbutyl | (S)-1,2-Dimethyl-2-hydroxypropylamino |
| 431 | 3-Hydroxy-3-methylbutyl | trans-4-Hydroxycyclohexylamino |
| 432 | 3-Hydroxy-3-methylbutyl | 1-Methansulfonylpiperidin-4-ylamino |
| 433 | 3-Hydroxy-3-methylbutyl | 1-Ethansulfonylpiperidin-4-ylamino |
| 434 | 4-Tetrahydropyranyl | Isobutylamino |
| 435 | 4-Tetrahydropyranyl | Isopropylamino |
| 436 | 4-Tetrahydropyranyl | trans-4-Hydroxy-4-methylcyclohexylamino |
| 437 | 4-Tetrahydropyranyl | Cyclopropylamino |
| 438 | 4-Tetrahydropyranyl | 2,2-Dimethylpropylamino |
| 439 | 1-Acetylpiperidin-4-yl | trans-4-Hydroxy-4-methylcyclohexylamino |
| 440 | 1-Acetylpiperidin-4-yl | 2,2-Dimethylpropylamino |
| 441 | 1-Acetylpiperidin-4-yl | Isopropylamino |
| 442 | 1-Acetylpiperidin-4-yl | Isobutylamino |
| 443 | 1-Acetylpiperidin-4-yl | Cyclopropylamino |
| 444 | 4-Tetrahydropyranyl | (R)-1,2-Dimethyl-2-hydroxypropylamino |
| 445 | 4-Tetrahydropyranyl | (S)-1,2-Dimethyl-2-hydroxypropylamino |
| 446 | 4-Tetrahydropyranyl | (S)-2-Hydroxy-1-methylethylamino |
| 447 | 4-Tetrahydropyranyl | (S)-1-Hydroxymethylpropylamino |
| 448 | 4-Tetrahydropyranyl | 1,1-Dimethyl-2-hydroxyethylamino |
| 449 | 4-Tetrahydropyranyl | 4-Tetrahydropyranylamino |
| 450 | 1-Acetylpiperidin-4-yl | 1,1-Dimethyl-2-hydroxyethylamino |
| 451 | 1-Acetylpiperidin-4-yl | 4-Tetrahydropyranylamino |
| 452 | 1-Acetylpiperidin-4-yl | (S)-1-Hydroxymethylpropylamino |
| 453 | 1-Acetylpiperidin-4-yl | (S)-1,2-Dimethyl-2-hydroxypropylamino |
| 454 | 4-Tetrahydropyranyl | trans-4-Hydroxycyclohexylamino |
| 455 | 1-Acetylpiperidin-4-yl | trans-4-Hydroxycyclohexylamino |
| 456 | 1-Methansulfonylpiperidin-4-yl | trans-4-Hydroxycyclohexylamino |
| 457 | 1-Methansulfonylpiperidin-4-yl | trans-4-Hydroxy-4-methylcyclohexylamino |
| 458 | 1-Methansulfonylpiperidin-4-yl | Isopropylamino |
| 459 | 4-Tetrahydropyranyl | 1-Methansulfonylpiperidin-4ylamino |
| 460 | 1-Acetylpiperidin-4-yl | 1-Methansulfonylpiperidin-4-ylamino |
| 461 | Isopropyl | 1-Acetylpiperidin-4-ylamino |
| 462 | Isopropyl | 1-Methansulfonylpiperidin-4-ylamino |
| 463 | Isopropyl | 1-(Isopropylsulfonyl)piperidin-4-ylamino |
| 464 | Isopropyl | 1-(Propylsulfonyl)piperidin-4-ylamino |
| 465 | Isopropyl | 1-(Butylsulfonyl)piperidin-4-ylamino |
| 466 | Isopropyl | 1-(Isobutyloxycarbonyl)piperidin-4-yl-amino |
| 467 | Isopropyl | 1-Butyrylpiperidin-4-ylamino |
| 468 | Ethyl | 1-Acetylpiperidin-4-ylamino |
| 469 | Ethyl | 1-Methansulfonylpiperidin-4-ylamino |
| 470 | Ethyl | 1-Ethansulfonylpiperidin-4-ylamino |
| 471 | Isopropyl | 1-Ethylcarbamoylpiperidin-4-ylamino |
| 472 | Isopropyl | 1-Propylcarbamoylpiperidin-4-ylamino |
| 473 | Isopropyl | 1-Isopropylcarbamoylpiperidin-4-ylamino |
| 474 | Isopropyl | 1-Ethansulfonylpiperidin-4-ylamino |
| 475 | Isopropyl | 1-Methoxycarbonylpiperidin-4-ylamino |
| 476 | Isopropyl | 1-Ethoxycarbonylpiperidin-4-ylamino |
| 477 | 1-Methansulfonylpiperidin-4-yl | Isopropylamino |
| 478 | 1-Ethansulfonylpiperidin-4-yl | Isopropylamino |

2. Verbindung nach Anspruch 1, die eine Verbindung der freien Base der Struktur 415 ist, oder ein pharmazeutisch akzeptables Salz derselben.
| | | |
|---|---|---|
| 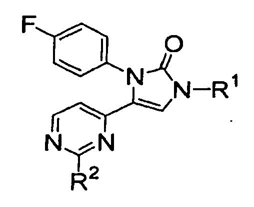 | | |
| Beispiel | R¹ | R² |
|---|---|---|
| 415 | Isopropyl | 2-Hydroxy-2-methylpropylamino |

3. Verbindung nach Anspruch 1, die eine Verbindung der freien Base der Struktur 417 ist, oder ein pharmazeutisch akzeptables Salz derselben.
| | | |
|---|---|---|
| 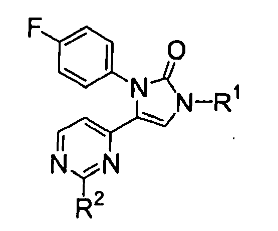 | | |
| Beispiel | R¹ | R² |
|---|---|---|
| 417 | 1,2-Dimethyl-2-hydroxypropyl | Isopropylamino |

4. Verbindung nach Anspruch 1, die eine Verbindung der freien Base der Struktur 436 ist, oder ein pharmazeutisch akzeptables Salz derselben.
| | | |
|---|---|---|
| 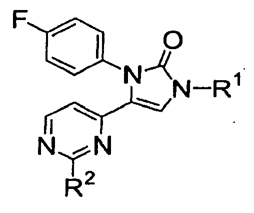 | | |
| Beispiel | R¹ | R² |
|---|---|---|
| 436 | 4-Tetrahydropyranyl | trans-4-Hydroxy-4-methyl cyclohexylamino |

5. Verbindung nach Anspruch 1, die eine Verbindung der freien Base der Struktur 460 ist oder ein pharmazeutisch akzeptables Salz derselben.
| | | |
|---|---|---|
| 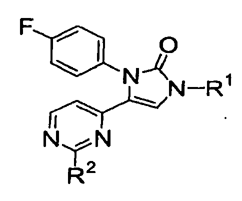 | | |
| Beispiel | R¹ | R² |
|---|---|---|
| 460 | 1-Acetylpiperidin-4-yl | 1-Methansulfonylpiperidin-4-ylamino |

6. Pharmazeutische Zusammensetzung, die die Verbindung nach einem der Ansprüche 1 bis 5 oder ein pharmazeutisch akzeptables Salz derselben und einen pharmazeutisch akzeptablen Träger umfasst.

7. Verwendung der Verbindung nach einem der Ansprüche 1 bis 5 oder eines pharmazeutisch akzeptablen Salzes derselben zur Herstellung eines p38 MAP-Kinase-Inhibitors.

8. Verwendung der Verbindung nach einem der Ansprüche 1 bis 5 oder eines pharmazeutisch akzeptablen Salzes derselben zur Herstellung eines Mittels zur Prophylaxe oder Behandlung einer Erkrankung, die aus der Gruppe von Arthritis, einer entzündlichen Darmerkrankung, einer entzündlichen Hauterkrankung, einer entzündlichen Atemwegserkrankung, einer entzündlichen Augenerkrankung, Nephritis, Hepatitis, einer systemischen entzündlichen Erkrankung, Schock, zerebrovaskulärer Verschlußkrankheit, einer ischämischen Herzerkrankung, Osteoporose, multipler Sklerose, Diabetes, einem malignen Tumor, Cachexia, Alzheimer-Krankheit, Parkinson-Krankheit, erworbenem Immunschwächesyndrom (AIDS), Arteriosklerose, disseminierter intravasaler Koagulation, Abstoßungs- und Transplantat-Wirt-Reaktionserkrankungen durch eine Organtransplantation ausgewählt ist.

9. Verbindung nach einem der Ansprüche 1 bis 5 oder pharmazeutisch akzeptables Salz derselben zur Verwendung bei der Prophylaxe oder Behandlung einer Erkrankung, die aus der Gruppe von Arthritis, einer entzündlichen Darmerkrankung, einer entzündlichen Hauterkrankung, einer entzündlichen Atemwegserkrankung, einer entzündlichen Augenerkrankung, Nephritis, Hepatitis, einer systemischen entzündlichen Erkrankung, Schock, zerebrovaskulärer Verschlußkrankheit, einer ischämischen Herzerkrankung, Osteoporose, multipler Sklerose, Diabetes, einem malignen Tumor, Cachexia, Alzheimer-Krankheit, Parkinson-Krankheit, erworbenem Immunschwächesyndrom (AIDS), Arteriosklerose, disseminierter intravasaler Koagulation, Abstoßungs- und Transplantat-Wirt-Reaktionserkrankungen durch eine Organtransplantation ausgewählt ist.

## Revendications

1. Composé choisi parmi les composés de base libre de structures 399 à 478, ou un sel pharmaceutiquement acceptable de celui-ci.
| | | |
|---|---|---|
| 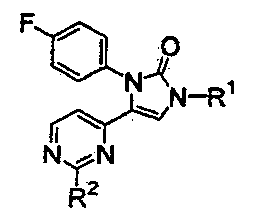 | | |
| | R¹ | R² |
|---|---|---|
| 399 | Ethyle | 1,1-Dioxotétrahydrothiophén-3-ylamino |
| 400 | Ethyle | trans-4-(Méthylcarbamoyl)cyclohexylamino |
| 401 | Ethyle | 1,5-Diméthyl-5-hydroxylhexylamino |
| 402 | Isopropyle | 1,5-Diméthyl-5-hydroxyhexylamino |
| 403 | Ethyle | cis-4-Hydroxy-4-méthylcyclohexylamino |
| 404 | Isopropyle | trans-4-Hydroxy-4-méthylcyclohexylamino |
| 405 | Isopropyle | trans-4-(1-Hydroxy-1-méthyléthyl)cyclohexylamino |
| 406 | Ethyle | trans-4-Hydroxy-4-méthylcyclohexylamino |
| 407 | Isopropyle | cis-4-Hydroxy-4-méthylcyclohexylamino |
| 408 | Ethyle | trans-4-(1-Hydroxy-1-méthyléthyl)cyclohexylamino |
| 409 | Ethyle | (S)-1,2-Diméthyl-2-hydroxypropylamino |
| 410 | Isopropyle | (S)-1,2-Diméthyl-2-hydroxypropylamino |
| 411 | Ethyle | 1,3-Diméthyl-3-hydroxybutylamino |
| 412 | Isopropyle | 1,3-Diméthyl-3-hydroxybutylamino |
| 413 | Isopropyle | 2-Mercapto-2-méthylpropylamino |
| 414 | Isopropyle | 1,1-Bishydroxyméthylpropylamino |
| 415 | Isopropyle | 2-Hydroxy-2-méthylpropylamino |
| 416 | Ethyle | 4-Pipéridylamino |
| 417 | 1,2-Diméthyl-2-hydroxypropyle | Isopropylamino |
| 418 | 1,2-Diméthyl-2-hydroxypropyle | cis-4-Hydroxy-4-méthylcyclohexylamino |
| 419 | 1,2-Diméthyl-2-hydroxypropyle | (S)-1,2-Diméthyl-2-hydroxypropylamino |
| 420 | 1,2-Diméthyl-2-hydroxypropyle | Trans-4-Hydroxy-4-méthylcyclohexylamino |
| 421 | 2-Hydroxy-1,1,2-triméthylpropyle | Isopropylamino |
| 422 | 2-Hydroxy-1,1,2-triméthylpropyle | Trans-4-hydroxy-4-méthylcyclohexylamino |
| 423 | 3-Hydroxy-3-méthylbutyle | Isopropylamino |
| 424 | 3-Hydroxy-3-méthylbutyle | 1,1-Diméthyl-2-hydroxyéthylamino |
| 425 | 3-Hydroxy-3-méthylbutyle | 2,2-Diméthyl-3-hydroxypropylamino |
| 426 | 3-Hydroxy-3-méthylbutyle | 1-Hydroxyméthylcyclopentylamino |
| 427 | 3-Hydroxy-3-méthylbutyle | trans-4-Hydroxy-4-méthylcyclohexylamino |
| 428 | 3-Hydroxy-3-méthylbutyle | 4-Tétrahydropyranylamino |
| 429 | 3-Hydroxy-3-méthylbutyle | (R)-1,2-Diméthyl-2-hydroxypropylamino |
| 430 | 3-Hydroxy-3-méthylbutyle | (S)-1,2-Diméthyl-2-hydroxypropylamino |
| 431 | 3-Hydroxy-3-méthylbutyle | trans-4-Hydroxycyclohexylamino |
| 432 | 3-Hydroxy-3-méthylbutyle | 1-Méthanesulfonylpipéridin4-ylamino |
| 433 | 3-Hydroxy-3-méthylbutyle | 1-Ethanesulfonylpipéridin-4-ylamino |
| 434 | 4-Tétrahydropyranyle | Isobutylamino |
| 435 | 4-Tétrahydropyranyle | Isopropylamino |
| 436 | 4-Tétrahydropyranyle | trans-4-Hydroxy-4-méthylcyclohexylamino |
| 437 | 4-Tétrahydropyranyle | Cyclopropylamino |
| 438 | 4-Tétrahydropyranyle | 2,2-Diméthylpropylamino |
| 439 | 1-Acétylpipéridin-4-yle | trans-4-Hydroxy-4-méthylcyclohexylamino |
| 440 | 1-Acétylpipéridin-4-yle | 2,2-Diméthylpropylamino |
| 441 | 1-Acétylpipéridin-4-yle | Isopropylamino |
| 442 | 1-Acétylpipéridin-4-yle | Isobutylamino |
| 443 | 1-Acétylpipéridin-4-yle | Cyclopropylamino |
| 444 | 4-Tétrahydropyranyle | (R)-1,2-Diméthyl-2-hydroxypropylamino |
| 445 | 4-Tétrahydropyranyle | (S)-1,2-Diméthyl-2-hydroxypropylamino |
| 446 | 4-Tétrahydropyranyle | (S)-2-hydroxy-1-méthyléthylamino |
| 447 | 4-Tétrahydropyranyle | (S)-1-Hydroxyméthylpropylamino |
| 448 | 4-Tétrahydropyranyle | 1,1-Diméthyl-2-hydroxyéthylamino |
| 449 | 4-Tétrahydropyranyle | 4-Tétrahydropyranylamino |
| 450 | 1-Acétylpipéridin-4-yle | 1,1-Diméthyl-2-hydroxyéthylamino |
| 451 | 1-Acétylpipéridin-4-yle | 4-Tétrahydropyranylamino |
| 452 | 1-Acétylpipéridin-4-yle | (S)-1-Hydroxyméthylpropylamino |
| 453 | 1-Acétylpipéridin-4-yle | (S)-1,2-Diméthyl-2-hydroxypropylamino |
| 454 | 4-Tétrahydropyranyle | trans-4-Hydroxycyclohexylamino |
| 455 | 1-Acétylpipéridin-4-yle | trans-4-Hydroxycyclohexylamino |
| 456 | 1-Méthanesulfonylpipéridin-4-yle | trans-4-Hydroxycyclohexylamino |
| 457 | 1-Méthanesulfonylpipéridin-4-yle | trans-4-Hydroxy-4-méthylcyclohexylamino |
| 458 | 1-Méthanesulfonylpipéridin-4-yle | Isopropylamino |
| 459 | 4-Tétrahydropyranyle | 1-Méthanesulfonylpipéridin4-ylamino |
| 460 | 1-Acétylpipéridin-4-yle | 1-Méthanesulfonylpipéridin4-ylamino |
| 461 | Isopropyle | 1-Acétylpipéridin-4-ylamino |
| 462 | Isopropyle | 1-Méthanesulfonylpipéridin-4-ylamino |
| 463 | Isopropyle | 1-(Isopropylsulfonyl)pipéridin-4-ylamino |
| 464 | Isopropyle | 1-(Propylsulfonyl)pipéridin-4-ylamino |
| 465 | Isopropyle | 1-(Butylsulfonyl)pipéridin-4-ylamino |
| 466 | Isopropyle | 1-(Isobutyloxycarbonyl)pipéridin-4-ylamino |
| 467 | Isopropyle | 1-Butyrylpipéridin-4-ylamino |
| 468 | Ethyle | 1-Acétylpipéridin-4-ylamino |
| 469 | Ethyle | 1-Méthanesulfonylpipéridin-4-ylamino |
| 470 | Ethyle | 1-Ethanesulfonylpipéridin-4-ylamino |
| 471 | Isopropyle | 1-Ethylcarbamoylpipéridin-4-ylamino |
| 472 | Isopropyle | 1-Propylcarbamoylpipéridin-4-ylamino |
| 473 | Isopropyle | 1-Isopropylcarbamoylpipéridin-4-ylamino |
| 474 | Isopropyle | 1-Ethanesulfonylpipéridin-4-ylamino |
| 475 | Isopropyle | 1-Méthoxycarbonylpipéridin-4-ylamino |
| 476 | Isopropyle | 1-Ethoxycarbonylpipéridin-4-ylamino |
| 477 | 1-Méthanesulfonylpipéridin-4-yle | Isopropylamino |
| 478 | 1-Ethanesulfonylpipéridin-4-yle | Isopropylamino |

2. Composé selon la revendication 1, qui est un composé de base libre de structure 415, ou un sel pharmaceutiquement acceptable de celui-ci.
| | | |
|---|---|---|
| 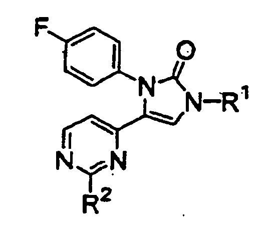 | | |
| Exemple | R¹ | R² |
|---|---|---|
| 415 | Isopropyle | 2-Hydroxy-2-méthylpropylamino |

3. Composé selon la revendication 1, qui est un composé de base libre de structure 417, ou un sel pharmaceutiquement acceptable de celui-ci.
| | | |
|---|---|---|
| 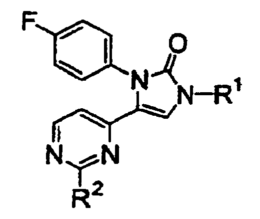 | | |
| Exemple | R¹ | R² |
|---|---|---|
| 417 | 1,2-Diméthyl-2-hydroxypropyle | Isopropylamino |

4. Composé selon la revendication 1, qui est un composé de base libre de structure 436, ou un sel pharmaceutiquement acceptable de celui-ci.
| | | |
|---|---|---|
| 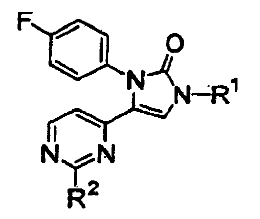 | | |
| Exemple | R¹ | R² |
|---|---|---|
| 436 | 4-Tétrahydropyranyle | trans-4-Hydroxy-4-méthylcyclohexylamino |

5. Composé selon la revendication 1, qui est un composé de base libre de structure 460, ou un sel pharmaceutiquement acceptable de celui-ci.
| | | |
|---|---|---|
| 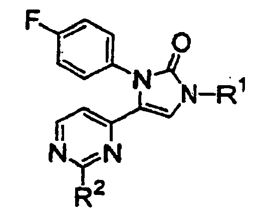 | | |
| Exemple | R¹ | R² |
|---|---|---|
| 460 | 1-Acétylpipéridin-4-yle | 1-Méthanesulfonylpipéridin-4-ylamino |

6. Composition pharmaceutique comprenant le composé selon l'une quelconque des revendications 1 à 5 ou un sel pharmaceutiquement acceptable de celui-ci, et un support pharmaceutiquement acceptable.

7. Utilisation d'un composé selon l'une quelconque des revendications 1 à 5 ou d'un sel pharmaceutiquement acceptable de celui-ci, pour la préparation d'un inhibiteur de la MAP kinase p38.

8. Utilisation du composé selon l'une quelconque des revendications 1 à 5 ou d'un sel pharmaceutiquement acceptable de celui-ci, pour la préparation d'un agent pour la prophylaxie ou le traitement d'une maladie choisie dans le groupe constitué par une arthrite, une maladie intestinale inflammatoire, une maladie dermique inflammatoire, une maladie respiratoire inflammatoire, une maladie optique inflammatoire, une néphrite, une hépatite, une maladie inflammatoire systémique, un choc, une maladie cérébrovasculaire, une cardiopathie ischémique, une ostéoporose, une sclérose en plaques, un diabète, une tumeur maligne, une cachexie, une maladie d'Alzheimer, une maladie de Parkinson, un syndrome d'immunodéficience acquise, une sclérose artérielle, un syndrome de coagulation intravasculaire disséminée, un rejet et des maladies greffon-contre-hôte par transplantation d'organe.

9. Composé selon l'une quelconque des revendications 1 à 5 ou sel pharmaceutiquement acceptable de celui-ci, pour une utilisation dans la prophylaxie ou le traitement d'une maladie choisie dans le groupe constitué par une arthrite, une maladie intestinale inflammatoire, une maladie dermique inflammatoire, une maladie respiratoire inflammatoire, une maladie optique inflammatoire, une néphrite, une hépatite, une maladie inflammatoire systémique, un choc, une maladie cérébrovasculaire, une cardiopathie ischémique, une ostéoporose, une sclérose en plaques, un diabète, une tumeur maligne, une cachexie, une maladie d'Alzheimer, une maladie de Parkinson, un syndrome d'immunodéficience acquise, une sclérose artérielle, un syndrome de coagulation intravasculaire disséminée, un rejet et des maladies greffon-contre-hôte par transplantation d'organe.
